# EUROPEAN PATENT SPECIFICATION

(11) **EP 3 833 268 B1**
(45) Date of publication and mention of the grant of the patent: **25.09.2024**
(21) Application number: 19847562.6
(22) Date of filing: 02.08.2019
(51) Int. Cl.: A61B 10/00, A61B 5/07, A61B 10/04, A61B 10/02, A61M 31/00

(54) **INGESTIBLE APPARATUS FOR COLLECTING SAMPLES IN THE GASTROINTESTINAL TRACT AND/OR DELIVERING HEALTH-RELATED SUBSTANCE THERETO**
EINNEHMBARE VORRICHTUNG ZUM SAMMELN VON PROBEN IM MAGEN-DARM-TRAKT UND ZUR ABGABE EINER GESUNDHEITSRELEVANTEN SUBSTANZ IN DIESEN
APPAREIL INGÉRABLE POUR COLLECTER DES ÉCHANTILLONS DANS LE TRACTUS GASTRO-INTESTINAL ET/OU POUR ADMINISTRER UNE SUBSTANCE LIÉE À LA SANTÉ À CELUI-CI

(30) Priority: 06.08.2018 US 201862714769 P; 15.05.2019 US 201916413026
(43) Date of publication of application: 16.06.2021
(73) Proprietor: Nimble Science Ltd., Calgary , AB T2P 3P2 (CA)
(72) Inventor: WANG, Gang, Calgary, Alberta T1Y 0B4 (CA)
(74) Representative: Patentanwälte Dr. Keller, Schwertfeger Partnerschaft mbB
(86) International application number: PCT/CA2019/051069
(87) International publication number: WO 2020/028978

(56) References cited:
- CA-A1- 2 961 307
- DE-B3- 102006 018 412
- DE-B3- 102006 018 412
- JP-A- 2003 325 438
- JP-A- 2003 325 438
- US-A- 4 036 214
- US-A- 4 481 952
- US-A1- 2008 194 912
- US-A1- 2018 070 857

## Description

### CROSS-REFERENCE TO RELATED APPLICATIONS

This application claims the benefit of US Provisional Patent Application Serial No. 62/714,769, filed August 06, 2018, and the US Patent Application Serial No. 16/413,026, filed May 15, 2019.

### FIELD OF THE DISCLOSURE

The present disclosure relates generally to an ingestible apparatus, and in particular to an ingestible apparatus and method for collecting samples of gastrointestinal-tract contents and/or delivering a health-related substance thereto.

### BACKGROUND

The gastrointestinal (GI) tract is a series of joined, hollow organs substantively in the form of a long and twisting tube from the mouth to the anus. The contents in the GI tract such as tissues, mucosal cells, microbiota, molecules, and the like contain information regarding the condition of the GI tract.

Samples of GI contents may be collected via stool sampling. However, the spatial and temporal information of the GI contents is poorly preserved in stool samples. Therefore, it is desirable in many situations to collect samples of GI contents directly from the interior of the GI tract by using a suitable sampling tool.

Different organs of the GI tract have varying degrees of accessibility. For example, small intestine is a deep and long organ which is difficult to access using transoral or transanal catheters. Therefore, various sampling tools and methods have been developed in prior art for accessing the GI tract and sampling therein.

Samples of contents in segments deep in the GI tract, particularly the small intestine, may be collected by aspiration using different types of tubes. For example, the academic article entitled "Device for obtaining samples of intestinal contents for microbiological and biochemical examinations," by Skala I, and Simacek J, and published in Gut, Apr. 1968, 9(2):246-248, teaches a hollow tube with a cylindrical head. The cylindrical head has an opening which may be switched on and off by air-suction force through the tube and a bias spring. After being transorally introduced into the GI tract, the tube head may be located by examining X-ray images. Samples of GI contents may be collected into the tube by using air-suction force.

By using such tubes with endoscopic-assisted aspiration or biopsy techniques, sampling in the upper GI tracts, colon, and even the proximal section of the small intestines may be effectively conducted as endoscopists may observe intraluminal images in real-time and collect the GI contents. However, these techniques are invasive. Moreover, these techniques are expensive and unsuitable for large-scale studies. Another disadvantage of these techniques is that the tubes are incapable of reaching the entire small-intestine.

US Patent No. 4,481,952 to Pawelec discloses a swallowable capsule for obtaining samples of gastric and/or intestinal contents, the capsule defining an inner-fluid receiving-chamber having an inlet port which is normally sealed off from the exterior of the capsule by way of a blocking means including a mass the properties of which change upon contact with the suitable fluids present in the digestion tract, causing the means to communicate the inlet port with the outside of the capsule allowing a sample of the fluid to be aspirated into the chamber. Located within the chamber is a closing means normally maintained in a flow-permitting position by a mass which glues the necessary parts of the closing means together in such a way that the parts cannot move, so that the action of the closing means is neutralized. This mass is responsive to contact with the suitable fluids of the alimentary canal to permit the closing means to operate and seal off the chamber so that the sample is secured from contamination during its further travel through the rest of the digestive tract.

The main advantage of this device is its low cost due to its structural simplicity. However, the sample collection heavily relies on the gravity, flow rate of GI contents, and the peristaltic forces of the specific part of the GI tract during the sampling time-period. The device is designed to collect samples from the intraluminal space, which comprises intestinal content which represent the luminal contents, but is generally not suitable for collecting the informationally rich mucosal and epithelial contents which reside within the mucosal layer of the GI tract and may carry more significant diagnostic values. Moreover, the device does not comprise any tracking module. Therefore, to track the location and sampling status of the device in-vivo, radiological scans such as scintigraphy or X-Ray scans are required which however are less preferred by patients due to irradiation concerns.

Other prior-art gastrointestinal-sampling techniques involve micro-electro-mechanical system (MEMS)-based capsules which utilizes an active mechanism to collect samples in the GI tract.

For example, the academic paper entitled "The study of a remote-controlled gastrointestinal drug delivery and sampling system," by Cui J, Zheng X, Hou W, Zhuang Y, Pi X, and Yang J, and published in Telemed J E Health, Sep. 2008, 14(7):715-9, doi: 10.1089/tmj.2007.0118, teaches a micro-machined capsule based on MEMS technology for diagnosing and treating gastrointestinal diseases. The microcapsule can carry out real-time drug release and gastrointestinal sampling in the gastrointestinal tract. According to the structural and metabolic characters of the gastrointestinal tract, the configuration of the microcapsule was designed as a cylinder. This nondigestible oral device can smoothly pass through the gastrointestinal tract for drug delivery and sampling of the intraluminal fluid. The working mechanism of the capsule was the mechanical movement mode of a piston, which was regulated through a MEMS calorific element. The action of drug delivery and gastrointestinal content sampling in the gastrointestinal tract was performed wirelessly. The remote control device can be connected with a computer through a serial port (RS-232), and it can be used in telemedicine applications. According to the authors, experimental results have indicated that the microcapsule can achieve drug delivery and the intraluminal fluid sample reliably.

Medimetrics Personalized Drug Delivery B.V. of Eindhoven, Netherlands has also developed a MEMS-based capsule called "Intellicap^{®}" (IntelliCap is a registered trademark of Koninklijke Philips N.V. of Amsterdam, Netherlands in European Union). The capsule integrates an electrical microactuator with multiple sensors for detecting modalities including pH and temperature and for estimating the location of the capsule in the GI tract. When the capsule arrives a desired location in the GI tract, the microactuator provides a one-way advancing to push drug out of a reservoir via an opening for targeted drug-delivery.

It is believed that the Intellicap^{®} may also be used for GI fluid sampling by redesigning the Intellicap^{®} to provide a one-way advancing with a reversed actuation-direction of the microactuator to cause a constant negative pressure in the reservoir for attracting GI fluid to flow therein via the same opening.

While such MEMS-based capsule technologies and microsystems have the advantage of accessing small intestines, the manufacturing cost thereof is expensive thereby impeding their clinical applicability. Miniaturization of these MEMS-based microsystems is difficult due to their complexity. Moreover, these technologies and microsystems have a low but critical risk of capsule retention in which case an endoscopic or even an open surgery would be required.

US4036214A describes fluid-collecting and microorganism-detecting devices, including a device as defined in the preamble of claim 1. In particular, a wick is disclosed having a frayed end, which is configured for transferring fluid down the wick by capillary action. A transfer means contacts bodily fluids in which microorganisms are present and such microorganisms flow by capillary action along the transfer means when the device is introduced into the body.

JP2003325438A describes a capsule medical device for insertion into a body cavity. In particular, a capsule having a capsule body is described, where a wire extends outside the capsule body and attaches to a brush. A rotating magnetic field is used to rotate a screw such that the brush is manipulated to collect cells such as cell from a mucous membrane.

DE102006018412B3 describes an endoscopy capsule for insertion into a body cavity. In particular, an endoscopy capsule having brush elements as a means for sampling a suspected tumorous alteration is described. The brush elements are arranged at an edge of cover. The sampling means are used to obtain a tissue smear. The endoscopy capsule includes a pair of brush elements placed below a retractable cover. The brush elements are mounted rotatably in opposite directions. The function of the protruding brush hairs is enabled by the rotation.

US4481952A describes a swallowable device for use in the examination of the alimentary canal. The device comprises a container having at least one inner, sample-receiving chamber and at least one opening defining an inlet port for said chamber; blocking means disposed on and cooperating with said container for maintaining said opening out of communication with the exterior of said container. The blocking means are comprised of a substance of a material having properties which enable it to dissolve following contact with predetermined contents of the alimentary canal, to cause said opening to be communicated with the exterior of the container, allowing a sample to pass from the alimentary canal into said inner chamber. The device also includes a sealing member movable to seal off said opening after a predetermined amount of time.

### SUMMARY

In view of the prior art, it is object of the present invention to provide an improved ingestible apparatus for collecting samples from a target area, in particular from a mucosal layer of a gastrointestinal tract of a human or mammal and/or for delivering a health-related substance thereto. This object is solved by an ingestible apparatus according to claim 1.

The claimed invention relates to an ingestible apparatus for collecting samples from a target area of a gastrointestinal (GI) tract of a human or mammal and/or for delivering a health-related substance thereto, the ingestible apparatus comprising:
a housing comprising at least one first port and a first compartment communicable with the at least one first port;
an actuation assembly for collecting the samples and/or discharging the health-related substance through the at least one first port; and
a delay structure for delaying the actuation assembly until the ingestible apparatus is within the target area. The actuation assembly comprises a brush, the brush comprising at least a stem and bristles coupled to the stem about a distal end thereof. The brush is configured to retract into the first compartment, and the bristles are configured to extend radially outward from the stem.

Embodiments disclosed herein generally relate to an ingestible apparatus and method for passively collecting gastrointestinal-content samples in a target area of the gastrointestinal (GI) tract of a human or a mammal (collectively denoted as a "user") and/or delivering a health-related substance thereto. The gastrointestinal-content samples to be collected may be collected from the intraluminal zone or from the mucosal wall layer lining, where samples of each will comprise fluid and solid components in varying percentages. For example, the mucosal layer will be denser in tissue and mucosal-layer samples than the sample from the luminal space.

In some embodiments, the ingestible apparatus disclosed herein comprises a wireless-signal transmitter and may be tracked by using a wireless-signal receiver outside the user's body.

According to one aspect of this disclosure, there is provided an ingestible apparatus for collecting samples from a target area of a gastrointestinal (GI) tract of a human or mammal and/or for delivering a health-related substance thereto. The ingestible apparatus comprises: a housing comprising at least one first port and at least a first compartment communicable with the at least one first port; an actuation assembly for changing the conditions of the first compartment for collecting the samples and/or discharging the health-related substance through the at least one first port; a delay structure for delaying the actuation assembly to change the conditions of the first compartment until the ingestible apparatus is at or about the target area; and a circuit structure for detecting arrival of the ingestible apparatus at the target area and for detecting an end of the sample collecting and/or the health-related-substance discharging.

In some embodiments, the changing conditions of the compartment is a change in volume. The change in volume related to the movement of the actuation assembly from a first to a second position.

In some embodiments, the circuit structure is configured for triggering a first signal-event when the arrival of the ingestible apparatus at the target area is detected, and triggering a second signal-event when the end of the sample collecting and/or the health-related-substance discharging is detected.

In some embodiments, the circuit structure comprises a radio-frequency (RF) signal transmitter for transmitting a RF signal when the first signal-event is triggered and stopping transmitting the RF signal when the second signal-event is triggered.

In some embodiments, the circuit structure is configured for detecting a start and an end of the actuation assembly movement from a first to a second position as the arrival of the ingestible apparatus at the target area and the end of the sample collecting and/or the health-related-substance discharging, respectively.

In some embodiments, the circuit structure comprises a first detection-circuit for detecting a gastrointestinal content in the GI tract as the start of the actuation assembly movement from a first to a second position of the first compartment.

In some embodiments, the first detection-circuit comprises a first set of spaced electrodes forming a first switch in an off configuration, the first switch being switchable to an on configuration when the first set of electrodes engage the gastrointestinal content.

In some embodiments, the delay structure comprises an isolation structure configured for isolating the first detection-circuit from the gastrointestinal content, the isolation structure dissolvable when contacting the gastrointestinal content.

In some embodiments, the actuation assembly comprises: a piston, at least a portion of the piston movably received in the housing; and a first actuation structure for actuating the piston along a first direction from a first to a second position to change the volume of the first compartment between a first volume and a second volume.

In some embodiments, the first actuation structure comprises one or more biased springs.

In some embodiments, the delay structure comprises a latch retaining the piston in a first position, the latch dissolvable when contacting the gastrointestinal contents.

In some embodiments, the latch is sandwiched between a retaining shoulder of the piston and the housing.

In some embodiments, the circuit structure comprises a second detection-circuit for detecting a movement of the piston as the end of the actuation from a first to a second position.

In some embodiments, the second detection-circuit comprises a second set of spaced electrodes forming a second switch in an off configuration, the second switch being switchable to an on configuration when the second set of electrodes engage an electrically conductive surface after the movement of the piston.

In some embodiments, the second detection-circuit comprises a Hall-effect sensor or a sliding rheostat having a slider coupled to the actuation structure.

In some embodiments, the second detection-circuit comprises a Hall-effect sensor or a sliding rheostat having a slider coupled to the piston.

In some embodiments, the isolation structure is further configured for isolating the latch and the second detection-circuit from the gastrointestinal contents.

In some embodiments, the isolation structure is a shell receiving therein the housing, the piston, the latch, the first actuation structure, and the circuit structure.

In some embodiments, the shell is coated with a dissolvable coating.

In some embodiments, the ingestible apparatus further comprises a sealable component for sealably closing the at least one first port to seal the first compartment.

In some embodiments, the first compartment is in communication with the at least one first port when the first compartment has a first volume; and the first compartment is fluidly disconnected from the at least one first port when the first compartment has a second volume.

In some embodiments, the ingestible apparatus further comprises a mucosal-layer brush extendable out of the first compartment for collecting the samples and retractable thereinto for storing the collected samples therein.

In some embodiments, the ingestible apparatus further comprises a mucosal-layer brush extendable out of the first compartment for delivering health-related substances into or underneath the mucosal layer at the target area as the device travels along the GI tract.

In some embodiments, the mucosal-layer brush is rotatable.

In some embodiments, the extension and/or retraction of the mucosal-layer brush is in the direction of the actuator assembly from its first to second position.

In some embodiments, the mucosal-layer brush comprises a stem and bristles. The bristles are spring loaded to extend radially outward from the stem upon movement of the actuation assembly from a first to a second position, such that the bristles push against the mucosal layer of the gastrointestinal tract.

In some embodiments, with movement of the actuation assembly, the bristles spring out with a radial force in a direction different to the direction of the mucosal-layer brush extension.

In some embodiments, the bristles comprise a fluid-absorbent material (i.e., a material suitable for absorbing gastrointestinal fluid) including but not limited to polyvinyl alcohol (PVA) to improve the collection of a mucosal sample from the mucosal layer of the GI tract in combination with the more fluid luminal sample from the intraluminal space.

In some embodiments, the bristles comprise a material for absorbing gastrointestinal content. The material, when absorbing fluid upon contact with the gastrointestinal contents, may change the properties of the bristles such that their contact with the mucosal layer is further improved. For example, the bristles of increased stiffness may be more adept at scratching off a mucosal sample from the mucosal layer, or contact with the mucosal layer may be improved.

In some embodiments, the mucosal-layer brush is coupled to a spring configured for compressing, extending and/or applying a torsion to the mucosal-layer brush. In one embodiment, the spring is coupled to the mucosal-layer brush for forcing the mucosal-layer brush against the mucosal layer to improve the adherence of the mucosal layer sample.

In some embodiments, the ingestible apparatus further comprises a string coupled to the shell or the housing.

In some embodiments, the string comprises marks for indicating a distance to the shell or the housing.

In some embodiments, the string comprises a biodegradable material.

In some embodiments, the string comprises a non-biodegradable material.

In some embodiments, the housing further comprises at least one second port and a second compartment in communication with the at least one second port for receiving therein the collected gastrointestinal contents; the first compartment is configured for containing therein the health-related substance; and the actuation assembly is configured for reducing the volume of the first compartment for discharging the health-related substance and for sealing the second compartment.

In some embodiments, the actuation assembly is configured for sealing the second compartment by isolating the second compartment from the at least one second port.

In some embodiments, a location of the second compartment is changeable; and the actuation assembly is configured for isolating the second compartment from the at least one second port by changing the location of the second compartment.

In some embodiments, the ingestible apparatus further comprises a second actuation structure for actuating the piston along a second direction opposite to the first direction.

In some embodiments, the ingestible apparatus further comprises a retaining block for retaining the second actuation structure at a biased configuration, the retaining block dissolvable in the gastrointestinal content so as to allow the second actuation structure to actuate the piston after the piston is actuated by the first actuation structure.

In some embodiments, the second actuation structure comprises one or more biased springs.

In some embodiments, the first compartment is configured for containing therein the health-related substance at an initial state and is reconfigurable for receiving therein the samples of gastrointestinal contents after the health-related substance therein is discharged.

In some embodiments, the at least one first port has a cross-sectional dimension allowing the health-related substance contained in the first compartment to form an interface at the at least one first port with a surface tension retaining the health-related substance in the first compartment.

According to one aspect of this disclosure, there is provided a method for collecting samples in a target area of a gastrointestinal (GI) tract and/or delivering a health-related substance thereto using a collection structure, the collection structure comprising a volume-changeable compartment therein. The method comprises: (i) allowing the collection structure to travel in the GI tract; (ii) triggering a first signal-event when the collection structure arrives the target area; (iii) moving the actuation assembly from a first to a second position to change the volume of the compartment for collecting the samples into the compartment or discharging the fluid therefrom; and (iv) triggering a second signal-event when the volume of the compartment is changed.

In some embodiments, said triggering the first signal-event when the collection structure arrives the target area comprises: disabling detection of a gastrointestinal content of the GI tract; and when the collection structure arrives the target area, enabling the detection of the gastrointestinal content and triggering the first signal-event when the gastrointestinal content is detected.

In some embodiments, said disabling detection of the gastrointestinal content of the GI tract comprises: isolating a first detection-structure from the gastrointestinal content using an isolation structure dissolvable in the gastrointestinal content.

In some embodiments, said disabling the detection of the gastrointestinal content further comprises: isolating the collection structure from the gastrointestinal content using the isolation structure.

In some embodiments, said isolating the collection structure from the gastrointestinal content using the isolation structure comprises: isolating the isolation structure from the gastrointestinal content using a coating dissolvable in a predefined area of the GI tract.

In some embodiments, the coating is an enteric-targeted coating.

In some embodiments, the coating is a pH-sensitive material which is dissolvable in an environment with a pH level greater than a predefined pH-level threshold;

In some embodiments, the pH-sensitive material is a pH-sensitive polymeric material.

In some embodiments, the coating is time-controllable which may have a predefined thickness and may dissolve gradually over a predefined (and sometimes prolonged) period of time.

In some embodiments, said enabling the detection of the gastrointestinal content comprises: allowing the isolation structure to dissolve in the gastrointestinal content so as to deform the isolation structure in the target area.

In some embodiments, said detecting the gastrointestinal content comprises: allowing a first switch to switch from an off configuration to an on configuration by the gastrointestinal content.

In some embodiments, the method further comprises: isolating a first set of spaced electrodes from the gastrointestinal content to configure the first switch to the off configuration; and said allowing the first switch to switch from the off configuration to the on configuration by the gastrointestinal content comprises: when the collection structure arrives the target area, allowing the first set of electrodes in contact with the gastrointestinal content thereby switching the first switch from the off configuration to the on configuration.

In some embodiments, said changing the volume of the compartment comprises: actuating a piston from a first to a second position to change the volume of the compartment.

In some embodiments, the method further comprises: retaining the piston at an initial position by using a latch dissolvable in the gastrointestinal content; and allowing the latch to dissolve in the gastrointestinal content so as to deform the latch in the target area.

In some embodiments, said triggering the second signal-event when the volume of the compartment is changed comprises: triggering the second signal-event when a movement of the piston is detected.

In some embodiments, said detecting the movement of the piston comprises: allowing a second switch to switch from an off configuration to an on configuration by the movement of the piston.

In some embodiments, the method further comprises: arranging a second set of spaced electrodes away from an electrically conductive surface and isolating the second set of electrodes from the gastrointestinal content to configure the second switch to the off configuration; and said allowing the second switch to switch from the off configuration to the on configuration by the movement of the piston comprises: allowing the movement of the piston to move the electrically conductive surface to engage the second set of electrodes thereby switching the second switch from the off configuration to the on configuration.

In some embodiments, the method further comprises: transmitting a radio-frequency (RF) signal when the first signal-event is triggered; and stopping the transmission of the RF signal when the second signal-event is triggered.

According to one aspect of this disclosure, there is provided an ingestible apparatus for gastrointestinal sampling and/or health-related-substance-delivery comprising: a housing comprising at least one first port and at least one second port; a piston movably received in the housing at a first position thereof, the piston defining in the housing a volume-changeable first compartment and a second compartment, the first compartment receiving therein a health-related substance and in communication with the at least one first port, the second compartment in communication with an exterior of the housing via the at least one second port when the piston is at the first position; a latch retaining the piston in the first position, the latch dissolvable in a gastrointestinal content of a gastrointestinal (GI) tract; and an actuation structure for actuating the piston from the first position to a second position in the housing thereby reducing the volume of the first compartment for discharging the health-related substance from the first compartment to a target area of the GI tract via the at least one first port, and isolating the second compartment from the exterior of the housing.

In some embodiments, the ingestible apparatus further comprises a shell dissolvable in the gastrointestinal content, the shell receiving therein the housing, the piston, the latch, and the actuation structure.

In some embodiments, the at least one first port has a cross-sectional dimension allowing the health-related substance received in the first compartment to form an interface in the at least one first port with a surface tension retaining the health-related substance in the first compartment.

In some embodiments, the actuation structure comprises one or more biased springs.

According to one aspect of this disclosure, there is provided an ingestible apparatus for gastrointestinal sampling and/or health-related-substance-delivery comprising: a housing comprising at least one port; a piston received in the housing and movable between a first position and a second position thereof, the piston comprising a sealable component and defining in the housing a volume-changeable compartment communicable with an exterior of the housing via the at least one port; a latch dissolvable in a gastrointestinal content of a gastrointestinal (GI) tract, the latch retaining the piston in the first position thereby configuring the compartment to a first volume for receiving therein a health-related substance and configuring the sealable component to sealably isolate the compartment from the exterior of the housing; a first actuation structure for actuating the piston from the first position to a second position thereby configuring the sealable component to enable the communication between the compartment and the exterior of the housing via the at least one port, and configuring the compartment to a second volume smaller than the first volume, for delivering the health-related substance from the compartment to a target area of the GI tract via the at least one port; and a second actuation structure for actuating the piston from the second position to the first position thereby configuring the compartment to the first volume for receiving samples collected from the target area of the GI tract and configuring the sealable component to sealably isolate the compartment from the exterior of the housing.

In some embodiments, the ingestible apparatus further comprises a shell dissolvable in the gastrointestinal content, the shell receiving therein the housing, the piston, the latch, and the first and second actuation structures.

In some embodiments, the ingestible apparatus further comprises a retaining block for retaining the second actuation structure at a biased configuration, the retaining block dissolvable in the gastrointestinal content.

In some embodiments, either one or both of the first and second actuation structures comprise one or more biased springs.

According to one aspect of this disclosure, there is provided an ingestible apparatus for sampling and/or health-related-substance-delivery comprising: a shell dissolvable in a gastrointestinal content of a gastrointestinal (GI) tract; a housing received in the shell, the housing comprising at least one port; a piston movably received in the housing and defining therein a volume-changeable compartment communicable with an exterior of the housing via the at least one port, the piston located at a first position in the housing thereby configuring the compartment to a first volume; a latch retaining the piston in the first position, the latch dissolvable in the gastrointestinal content; an actuation structure for actuating the piston from the first position to a second position in the housing thereby configuring the compartment to a second volume for collecting samples from a target area of the GI tract into the compartment and/or delivering a health-related substance from the compartment to the target area via the at least one port; and a circuit structure comprising a power-source component, a first detection-circuit for detecting the gastrointestinal content, a second detection-circuit for detecting a position of the piston, and a radio-frequency (RF) signal transmitter electrically coupled to the power-source component and the first and second detection-circuits for transmitting at least one RF signal indicative of a state of the ingestible apparatus. The first detection-circuit is at a first configuration and is reconfigurable to a second configuration when in contact with the gastrointestinal content; the second detection-circuit is at a third configuration when the piston is at the first position and is reconfigurable to a fourth configuration when the piston is at the second position; and a combination of the configurations of the first and second detection-circuits determines values of a set of properties of the at least one RF signal for indicating the state of the ingestible apparatus.

According to one aspect of this disclosure, there is provided an ingestible apparatus for sampling and/or health-related-substance-delivery comprising: a housing comprising a volume-changeable first compartment communicable with at least one port; a piston, at least a portion of the piston movably received in the housing; a latch retaining the piston in a first position, the latch dissolvable when contacting a gastrointestinal content of a gastrointestinal (GI) tract; a first actuation structure for actuating the piston along a first direction from a first position to a second position, for collecting samples from a target area of the GI tract into the first compartment and/or delivering a health-related substance from the first compartment thereto via the at least one port; a circuit structure comprising a power-source component, a first detection-circuit for detecting the gastrointestinal content, a second detection-circuit for detecting a position-change of the piston, and a radio-frequency (RF) signal transmitter electrically coupled to the power-source component and the first and second detection-circuits for transmitting a signal at least between the detection of the gastrointestinal content by the first detection-circuit and the detection of the position-change of the piston by the second detection-circuit; and an isolation structure isolating at least the first and the second detection-circuits from the gastrointestinal content, the isolation structure dissolvable in the gastrointestinal content.

### BRIEF DESCRIPTION OF THE DRAWINGS

FIG. 1A is a cross-sectional view of an ingestible apparatus for delivering a health-related substance through the gastrointestinal (GI) tract of a human or mammal (collectively denoted as a "user") to a target area therein, according to some embodiments of this disclosure;
FIG. 1B is a circuit diagram of the ingestible apparatus shown in FIG. 1A;
FIG. 2 is a cross-sectional view of the ingestible apparatus shown in FIG. 1A in the GI tract, wherein an enteric-targeted coating thereof is substantively deformed;
FIG. 3A is a cross-sectional view of the ingestible apparatus shown in FIG. 1A, wherein a shell of the ingestible apparatus is substantively deformed;
FIG. 3B is a circuit diagram of the ingestible apparatus shown in FIG. 3A;
FIG. 4A is a cross-sectional view of the ingestible apparatus shown in FIG. 1A, wherein the ingestible apparatus discharges the health-related substance to the target area;
FIG. 4B is a circuit diagram of the ingestible apparatus shown in FIG. 4A;
FIG. 5 shows the output of a tracking device monitoring the ingestible apparatus shown in FIG. 1A during a health-related-substance-delivery process;
FIG. 6 shows the output of a tracking device monitoring the ingestible apparatus shown in FIG. 1A during a health-related-substance-delivery process, according to some embodiments of this disclosure;
FIG. 7 is a cross-sectional view of an ingestible apparatus for collecting gastrointestinal-content samples from the mucosal layer in a target area of the GI tract of a user, according to some embodiments of this disclosure;
FIG. 8 is a cross-sectional view of the ingestible apparatus shown in FIG. 7, wherein a coating of the ingestible apparatus is substantively deformed;
FIG. 9 is a cross-sectional view of the ingestible apparatus shown in FIG. 7, wherein a shell of the ingestible apparatus is substantively deformed thereby allowing the ingestible apparatus to collect samples from the mucosal layer of the GI tract;
FIG. 10 is a cross-sectional view of the ingestible apparatus shown in FIG. 7, wherein a compartment of the ingestible apparatus is sealed with the collected samples from the mucosal layer received therein;
FIG. 11 is a cross-sectional view of an ingestible apparatus for collecting samples in a target area of the GI tract of a user, according to some embodiments of this disclosure;
FIG. 12 is a cross-sectional view of the ingestible apparatus shown in FIG. 11, wherein a coating of the ingestible apparatus is substantively deformed;
FIG. 13 is a cross-sectional view of the ingestible apparatus shown in FIG. 11, wherein a shell of the ingestible apparatus is substantively deformed thereby allowing the ingestible apparatus to collect samples;
FIG. 14 is a cross-sectional view of the ingestible apparatus shown in FIG. 11, wherein a compartment of the ingestible apparatus is sealed with the collected mucosal-layer samples received therein;
FIG. 15 is a cross-sectional view of an ingestible apparatus for collecting samples from a target area of the GI tract of a user and then delivering a health-related substance thereto, according to some embodiments of this disclosure;
FIG. 16 is a cross-sectional view of the ingestible apparatus shown in FIG. 15, wherein a coating and a shell of the ingestible apparatus are substantively deformed thereby allowing the ingestible apparatus to collect samples to a second compartment thereof and then discharge the health-related substance from a first compartment thereof;
FIG. 17 is a cross-sectional view of the ingestible apparatus shown in FIG. 15, wherein the collected samples are sealed in the second compartment and the health-related substance has been discharged from the first compartment;
FIG. 18 is a cross-sectional view of an ingestible apparatus for delivering a health-related substance through the GI tract of a user to a target area therein and then collecting samples therefrom, according to some embodiments of this disclosure, wherein the ingestible apparatus comprises a sampling actuation-structure having two biased springs;
FIG. 19 is a cross-sectional view of the ingestible apparatus shown in FIG. 18, wherein a shell of the ingestible apparatus is substantively deformed thereby allowing the ingestible apparatus to discharge the health-related substance from a compartment and then collect samples into the compartment;
FIG. 20 is a cross-sectional view of the ingestible apparatus shown in FIG. 18, wherein the health-related substance has been discharged from the compartment;
FIG. 21A is a cross-sectional view of the ingestible apparatus shown in FIG. 18, wherein the samples are collected into the compartment;
FIG. 21B is a circuit diagram of the ingestible apparatus shown in FIG. 21A;
FIG. 22 is a cross-sectional view of the ingestible apparatus shown in FIG. 18, wherein the collected samples are sealed in the compartment;
FIG. 23 is a cross-sectional view of an ingestible apparatus for delivering a health-related substance through the GI tract of a user to a target area therein and then collecting samples therefrom, according to some embodiments of this disclosure, wherein the ingestible apparatus comprises a sampling actuation-structure having one biased spring;
FIG. 24 is a cross-sectional view of the ingestible apparatus shown in FIG. 23, wherein a shell of the ingestible apparatus is substantively deformed thereby allowing the ingestible apparatus to discharge the health-related substance from a compartment and then collect samples into the compartment;
FIG. 25 is a cross-sectional view of the ingestible apparatus shown in FIG. 23, wherein the health-related substance has been discharged from the compartment;
FIG. 26 is a cross-sectional view of the ingestible apparatus shown in FIG. 23, wherein the samples are collected into the compartment;
FIG. 27 is a cross-sectional view of the ingestible apparatus shown in FIG. 23, wherein the collected samples are sealed in the compartment;
FIG. 28 is a cross-sectional view of an ingestible apparatus for delivering a health-related substance through the GI tract of a user to a target area therein and then collecting mucosal-layer samples therefrom, according to some embodiments of this disclosure;
FIG. 29 is a cross-sectional view of the ingestible apparatus shown in FIG. 28, wherein a shell of the ingestible apparatus is substantively deformed thereby allowing the ingestible apparatus to discharge the health-related substance from a compartment and then collect mucosal-layer samples into the compartment;
FIG. 30 is a cross-sectional view of the ingestible apparatus shown in FIG. 28, wherein the health-related substance has been discharged from the compartment;
FIG. 31 is a cross-sectional view of the ingestible apparatus shown in FIG. 28, wherein the mucosal-layer samples are collected by a mucosal-layer brush;
FIG. 32 is a cross-sectional view of the ingestible apparatus shown in FIG. 28, wherein the collected mucosal-layer samples are sealed in the compartment;
FIGs. 33A and 33B are cross-sectional views of a first switch of an ingestible apparatus configured in an off and an on configurations, respectively, according to some embodiments of this disclosure;
FIGs. 34A and 34B are cross-sectional views of a first switch of an ingestible apparatus configured in an off and an on configurations, respectively, according to some alternative embodiments of this disclosure;
FIGs. 35A and 35B are cross-sectional views of a first switch of an ingestible apparatus configured in an off and an on configurations, respectively, according to yet some alternative embodiments of this disclosure;
FIGs. 36A and 36B are cross-sectional views of a second switch of an ingestible apparatus configured in an off and an on configurations, respectively, according to some embodiments of this disclosure;
FIGs. 37A and 37B are cross-sectional views of a second switch of an ingestible apparatus configured in an off and an on configurations, respectively, according to some alternative embodiments of this disclosure;
FIG. 38 is a block diagram of a wearable tracking device for tracking an ingestible apparatus in a sampling and/or health-related-substance-delivery process, according to some embodiments of this disclosure;
FIG. 39 is a schematic diagram showing a user ingesting a plurality of ingestible apparatuses and wearing the wearable tracking device shown in FIG. 38 for tracking the plurality of ingestible apparatuses in a sampling and/or health-related-substance-delivery process;
FIG. 40 shows the output of the tracking device shown in FIG. 38 during the sampling and/or health-related-substance-delivery process shown in FIG. 39;
FIG. 41 is a flowchart showing the steps of a monitoring process for monitoring the sampling and/or health-related-substance-delivery process shown in FIG. 39 with X-Ray confirmation;
FIG. 42 is a schematic diagram showing an ingestible apparatus, according to in some embodiments of this disclosure, wherein the ingestible apparatus comprises a string coupled to a shell thereof for monitoring the travelling of the ingestible apparatus in a GI tract.
FIG. 43 is a cross-sectional view of an ingestible apparatus for delivering a health-related substance through the GI tract of a user to a target area therein and then collecting mucosal-layer samples therefrom, according to some embodiments of this disclosure, wherein the ingestible apparatus comprises a mucosal-layer brush rotatable with a piston mandrel;
FIG. 44 is a cross-sectional view of an ingestible apparatus for delivering a health-related substance through the GI tract of a user to a target area therein and then collecting mucosal-layer samples therefrom, according to some embodiments of this disclosure, wherein the ingestible apparatus comprises a mucosal-layer brush rotatable with a rotatable sleeve on a piston mandrel;
FIG. 45 is a cross-sectional view of an ingestible apparatus for delivering a health-related substance through the GI tract of a user to a target area therein and then collecting mucosal-layer samples therefrom, according to some embodiments of this disclosure, wherein the ingestible apparatus does not comprises a dissolvable shell;
FIG. 46 is a cross-sectional view of an ingestible apparatus for collecting samples from a target area of the GI tract of a user and then delivering a health-related substance thereto, according to some embodiments of this disclosure;
FIG. 47 is a cross-sectional view of the ingestible apparatus shown in FIG. 46, wherein a coating and a shell of the ingestible apparatus are substantively deformed;
FIG. 48 is a cross-sectional view of the ingestible apparatus shown in FIG. 46, wherein bristles of a brush of the ingestible apparatus are extended out of a housing thereof for collecting samples; and
FIG. 49 is a cross-sectional view of the ingestible apparatus shown in FIG. 46, wherein the bristles of the brush and the collected samples are retrieved into the housing and sealed in a compartment thereof.

### DETAILED DESCRIPTION

Embodiments disclosed herein relate to an ingestible apparatus for delivering health-related substance comprising one or more active compounds to a target area of a gastrointestinal (GI) tract of a human or mammal (collectively denoted as a "user") and/or collecting gastrointestinal-content samples therefrom.

The ingestible apparatus comprises at least one first compartment communicable with one or more first ports, and an actuation assembly for adjusting the conditions of the first compartment for above-described purposes. For example, in various embodiments, the actuation assembly may move from a first position to a second position thereby to (a) increase the volume of the first compartment to receive samples collected from the target area; or (b) reduce volume of the first compartment to discharge a health-related substance therein into the target area; or (c) reduce volume of the first compartment to discharge the health-related substance therein into the target area and then increase the volume of the first compartment to receive samples collected from the target area.

In an alternate embodiment, the actuation assembly may move from a first position to a second position thereby adjusting the conditions of the first compartment by mechanisms other than changing the volume of the compartment. For example, the movement may close or seal the fluidly communicable port of the compartment to collect a gastrointestinal sample, or may expose a previously closed or sealed port to discharge the health-related substance.

In some embodiments, the ingestible apparatus may further comprise a second compartment in communication with one or more second ports. The first compartment may contain therein the health-related substance. The ingestible apparatus may receive samples collected from the target area into the second compartment and then reduce volume of the first compartment to discharge the health-related substance therein into the target area.

In some embodiments, the actuation assembly may comprise an actuation structure for actuating a piston to change the volume of the first compartment. The piston may be movable between the first position and a second position; and wherein one of the first and second positions is away from the at least one first port and the other one of the first and second positions is adjacent the at least one first port.

In some embodiments, the actuation structure may be one or more loaded, biased, or otherwise energy-stored springs.

In some embodiments, the ingestible apparatus may comprise a sealing mechanism for sealing the first and/or the second compartments at the end of the action of the actuation assembly. The sealing mechanism may be a sealing component for closing the first and/or second ports, or may be an action of the actuation assembly that isolates the first and/or second compartment from the corresponding first and/or second port, or may be the first and/or second ports having a sufficiently small cross-sectional dimension to form a liquid interface therein with a surface tension sufficient for retaining the health-related substance in the corresponding compartment.

In some embodiments, the ingestible apparatus may comprise a mucosal-layer brush for collecting mucosal-layer samples.

In some embodiments, the mucosal-layer brush may alternatively or additionally be used to deliver a health-related-substance to the mucosal layer.

In some embodiments, the mucosal-layer brush may be retractable into the first compartment and may be rotatable for providing an improved sampling performance.

In some embodiments, the ingestible apparatus may comprise a fluid-absorbent material such as hydrophilic fibers for facilitating the sampling of gastrointestinal content.

In some embodiments, the mucosal-layer brush may comprise bristles to either collect gastrointestinal content or to deliver health-related substances.

In some embodiments, the bristles may comprise a fluid-absorbent material (i.e., a material suitable for absorbing gastrointestinal fluid) such as hydrophilic fibers, e.g., polyvinyl alcohol (PVA), to improve the collection of a mucosal sample from the mucosal layer of the GI tract in combination with the more fluid luminal sample from the intraluminal space.

The ingestible apparatus may also comprise a delay structure for delaying the action of the actuation assembly until the ingestible apparatus arrives or is about to arrive the target area. Such a delay structure may provide a regional delay (i.e., providing a delay until the ingestible apparatus has arrived a predefined area) or a time delay (i.e., providing a delay until a predefined period of time has passed).

In some embodiments, the delay structure may comprise a dissolvable component retaining the actuation assembly at an initial state which may gradually dissolve and/or substantive deform after a regional delay and/or a time delay. In some embodiments, the delay structure may comprise a dissolvable shell enclosing other components therein. In some embodiments, the delay structure may further comprise a dissolvable coating for providing a regional delay.

In some embodiments, the ingestible apparatus may further comprise a circuit structure for monitoring a sampling and/or health-related-substance-delivery process of the ingestible apparatus by detecting the arrival of the ingestible apparatus at the target area and by detecting an end of the process.

In some embodiments, the circuit structure is configured for detecting a start and an end of the volume-changing of the first compartment (which may correspond to a start and an end of the action of the actuation assembly, for example from a first position to a second position) as the arrival of the ingestible apparatus at the target area and the end of the sample collecting and/or the health-related-substance discharging, respectively.

In some embodiments, the circuit structure may use a timer to record the time instant of the occurrence of each state of the ingestible apparatus and stores the recorded time instants in a memory.

In some embodiments, the circuit structure may comprise a power-source component and a radio-frequency (RF) signal transmitter for transmitting a RF signal at least between the start and the end of the volume-changing of the first compartment. The circuit structure may transmit different RF signals for indicating various states of the ingestible apparatus during the sampling and/or health-related-substance-delivery process. A monitoring device may be used outside the user's body for monitoring the sampling and/or health-related-substance-delivery process by monitoring the RF signals. Herein, a RF signal has a set of unique property settings such as amplitude, frequency, modulation, coding, and/or the like. Two RF signals are different in at least one property setting. Moreover, no RF signal transmission may be considered a special RF signal which has a zero (0) amplitude.

In some embodiments, the circuit structure comprises a first detection circuit for detecting gastrointestinal content as an indication of the start of the action of the actuation assembly. In these embodiments, the first detection circuit may be disabled until the ingestible apparatus arrives or is about to arrive the target area. In these embodiments, the first detection circuit may be isolated from the gastrointestinal content until the ingestible apparatus arrives or is about to arrive the target area. In some embodiments, the first detection circuit may comprise a first set of spaced electrodes engageable with the gastrointestinal content (which is generally electrically conductive) forming a first switch.

In some embodiments, the circuit structure comprises a second detection circuit for detecting the changing conditions of the first and/or second compartment as an indication of the end of the action of the actuation assembly. In the embodiments wherein the actuation assembly comprises a piston, the second detection circuit may detect the position-change or movement of the piston as an indication of the volume-change of the first and/or second compartment. In some embodiments, the second detection circuit may comprise a second set of spaced electrodes engageable with an electrically conductive surface forming a second switch, the second set of electrodes and the electrically conductive surface are movable relative to each other in accordance with the action of the actuation assembly.

In some embodiments, the first switch is electrically coupled to the power-source component and the RF-signal transmitter in series; wherein the second switch electrically coupled to the power-source component and in parallel with the second switch and the RF-signal transmitter.

In some embodiments, the RF-signal transmitter may be a radio-frequency identification (RFID) chip with an antenna.

In some embodiments, the ingestible apparatus may further comprise a string with marks for determining the position of the ingestible apparatus during a sampling and/or health-related-substance-delivery process. The string may be made of a dissolvable material.

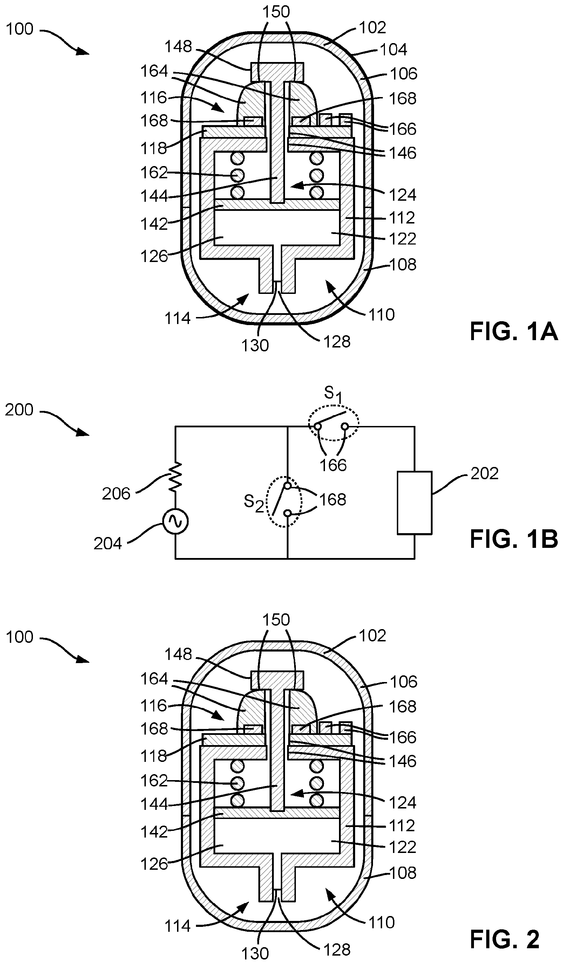

Turning now to FIG. 1A, an ingestible apparatus according to some embodiments of this disclosure is shown and is generally identified using reference numeral 100. In these embodiments, the ingestible apparatus 100 is an ingestible health-related-substance-delivery apparatus in the form of a capsule and may be used for delivering a health-related substance through the GI tract of a user to a target area therein.

In various embodiments, the target area may not need to be highly precise. As those skilled in the art understand, the GI tract is a series of joined, hollow organs substantively in the form of a long and twisting tube from the mouth to the anus. In some embodiments, the target area may be anywhere within a particular organ along the GI tract. In some embodiments, the target area may be a specific range within a particular organ along the GI tract. Generally, the target area may be defined by a doctor or a specialist with a certain precision as needed. Examples of the target area include but not limited to esophagus, stomach, small intestine, and large intestine of the gastrointestinal tract.

Accordingly, some characteristics of the ingestible apparatus 100 (described in more detail below) may be customized such as by selecting a suitable one from a plurality of pre-manufactured ingestible apparatuses 100 with various characteristics thereof.

In some embodiments, a plurality of pre-manufactured ingestible apparatuses 100 with various characteristics are provided as a kit. The ingestible apparatuses 100 of the kit may have different settings such as different target areas, different functionalities (e.g., sampling and/or health-related-substance-delivery), and/or different health-related substances for delivery.

Depending on the characteristics of the patient or user, including the presence of medical conditions or medication use affecting motility rate, or depending on the intended target area, one or more ingestible apparatuses may be selected from the kit and provided to the user. Alternatively, the user may ingest multiple, including all ingestible apparatuses provided within the kit, to increase the likelihood that at least one device samples gastrointestinal content or delivers health-related-substance to the intended target area. In another embodiment, the user ingest multiple ingestible apparatus of various characteristics as provided in a kit to sample gastrointestinal contents and/or deliver a health-related substance at multiple points along the GI tract.

In another embodiment, a user may ingest one of the plurality of ingestible apparatuses 100 of the kit for collecting gastrointestinal samples. Based on the processing and analysis results of the collected samples, the user may further ingest one or more ingestible apparatuses 100 for sampling and/or for delivering necessary health-related substances to specific target area(s).

In the example shown in FIG. 1A, the ingestible apparatus 100 is for delivering a health-related substance to a target enteric area and comprises a dissolvable shell 102 coated with an enteric-targeted coating 104 and a delivery structure 110 enclosed in the dissolvable shell 102. Herein, the health-related substance may comprise one or more active compounds such as one or more pharmaceutical compounds, one or more probiotics, one or more prebiotics, one or more microbiomes, and/or the like.

In various embodiments, the one or more active compounds may be manufactured compounds, or compounds obtained directly or indirectly from human or animal. For example, the health-related substance may comprise active compounds obtained from the user or from another person (referred to as a donor), or a group of persons wherein the obtained active compounds may be processed, modified, and/or combined, and then directly delivered back to the user.

The health-related substance may be in the form of a fluid, a powder, a solid substance, or a mixture thereof. The health-related substance may be a sample of stool, or other GI contents from small intestine or large intestine, previously collected from the same user, or from a donor, which may be used to improve or treat the microbiome of the user or to reestablish a previous microbiome prior to treatment.

While the health-related substance may be in various forms, the following description uses a liquid-form health-related substance as an example for ease of description.

The enteric-targeted coating 104 is made of a suitable ingestible material which substantively maintains its integrity until arriving a predefined area (before the target enteric area) in which the coating 104 starts to dissolve at a significant rate and is sufficiently deformed after approximately a predefined period of time for exposing the shell 102 to the digestive system.

For example, the coating 104 may be formed by an ingestible material dissolvable at a fast rate in an environment (such as the stomach) in which the gastrointestinal content (generally comprising gastrointestinal fluid) has a pH value greater than a predefined pH-level threshold and dissolvable at a slow rate or even undissolvable in an environment (such as the GI tract before and in the stomach) in which the gastrointestinal content has a pH value less than the predefined threshold, thereby giving rise to a regional dissolution or regional delay.

In some embodiments, the pH-sensitive material is a pH-sensitive polymeric material.

In some embodiments, the coating is time-controllable which may have a predefined thickness and may dissolve gradually over a predefined (and sometimes prolonged) period of time.

With such a regional delay, the shell 102 is protected from being dissolved until the ingestible apparatus 100 reaches the stomach when the coating 104 is sufficiently deformed, thereby preventing the failure that the ingestible apparatus 100 releases the health-related substance before reaching the target enteric area.

In some embodiments, the coating 104 may be formed by an ingestible material that may dissolve after a predetermined time period. The predetermined time period may be longer than a typical gastric transit time (i.e. the time required for a capsule device to pass stomach and reach small intestine) thereby preventing the failure that the ingestible apparatus 100 may otherwise release the health-related substance before reaching the target enteric area such as small intestine.

In some embodiments, the coating 104 may be formed by an ingestible material that may be degraded by specific enzymes predominantly existing in the colon, in which the intestinal content has unique enzymatic ability produced by the colonic micro flora, thereby giving rise to a regional specific delay. With such a regional delay, the shell 102 is protected from being dissolved until the ingestible apparatus 100 reaches the stomach when the coating 104 is sufficiently deformed, thereby preventing the failure that the ingestible apparatus 100 may otherwise release the health-related substance before reaching the target enteric area.

In some embodiments, the ingestible apparatus 100 may not comprise the coating 104, thereby leaving the shell 102 directly exposed to the gastrointestinal content after oral administration and dissolves quickly in the stomach and the ingestible apparatus 100 starts to release the health-related substance in the stomach, or to obtain luminal or mucosal samples in the stomach (described in more detail later).

The shell 102 is made of a suitable ingestible and dissolvable material such as gelatin or other suitable animal proteins, carrageenans, modified starch, cellulose, and/or other plant polysaccharides or derivatives that are often used in making conventional ingestible capsules. Preferably, the shell is a hard-shelled capsule made of two pieces including a shell cap 106 and a shell body 108 coupled together and sealed at their interface by using suitable capsule manufacturing technologies such as the conventional capsule manufacturing technologies known in the art.

The shell 102 isolates the components therein from the digestive system and is dissolvable at a predetermined rate (determined by the characteristics of the material thereof) when in contact with the digestive system. By selecting a thickness thereof, the shell 102 provides a predetermined time delay such that its integrity is substantively maintained until the ingestible apparatus 100 arrives the target area (within approximately a predetermined period of time) when the shell 102 is sufficiently deformed for allowing the health-related substance therein to release.

In some embodiments, the shell 102 may be similar to that of the ingestible PillCam^{™} Patency Capsule (PillCam is a trademark of GIVEN IMAGING LTD. of ISRAEL).

Enclosed in the shell 102, the delivery structure 110 in these embodiments comprises a housing 112 having a front end 114 and a rear end 116 with a circuit structure 118 coupled to the rear end 116 thereof.

The housing 112 may comprise two portions coupled together via suitable coupling means such as threading, glue, screws, an/or the like to form a chamber 122 and receive therein a piston 124. The piston 124 is movable in the chamber 122 between a front position adjacent the front end 114 and a rear position adjacent the rear end 116 thereof. The piston 124 defines a compartment 126 between the piston 124 and the front end 114 for receiving therein the health-related-substances. The compartment 126 comprises one or more substance-release ports 128 on the housing 112 (e.g., one substance-release port 128 at the front end 114 of the housing 112 in the example shown in FIG. 1A) for releasing the health-related substance.

In these embodiments, each of the one or more substance-release ports 128 has a small cross-sectional dimension allowing the health-related substance in the compartment 126 to form a liquid surface or interface 130 with a surface-tension force (and optionally the friction of the sidewall of the substance-release port 128) that is sufficient for retaining the health-related substance in the compartment 126.

The piston 124 comprises a piston plate 142 and a piston mandrel 144 extending rearwardly from the piston plate 142 out of the housing 112 to an end piece 148 via the aligned access holes 146 respectively on the rear end 116 of housing 112 and on the circuit structure 118. The end piece 148 has an extended diameter thereby forming a circumferential retaining-shoulder 150 facing the housing 112. The end piece 148 is made of an electrically conductive material such a copper or alternatively the circumferential retaining-shoulder 150 is coated, printed, deposited, or otherwise coupled with a layer of the electrically conductive material for forming an electrical switch (described in more detail later).

When the ingestible apparatus 100 is in an initial state T_{A}, the piston 124 is located at the rear position adjacent the rear end 116 of the chamber 122 and is retained thereto by a dissolvable latch 164 engaging the circumferential retaining-shoulder 150 of the end piece 148 and the circuit structure 118, thereby configuring the compartment 126 to its maximum volume. An actuation structure comprising a biased spring 162 engages the piston 124 for moving the piston 124 to the front position when the dissolvable latch 164 is dissolved. The actuation structure and the piston thus form an actuation assembly for changing the volume of the compartment 126.

In the example shown in FIG. 1A, the biased spring 162 is a compressed extendable-spring located in the chamber 122 between the rear end 116 and the piston 124. The spring 162 is extendable to "push" the piston 124 towards the front end 114 to reduce the volume of the compartment 126 and discharge the health-related substance therein. In some alternative embodiments, the biased spring 162 may be an extended compressible-spring located in the chamber 122 between the front end 114 and the piston 124 (i.e., in the compartment 126). The spring 162 is retractable to "pull" the piston 124 towards the front end 114 to reduce the volume of the compartment 126 and discharge the health-related substance therein.

The circuit structure 118 comprises a sensing circuitry (not shown) electrically connected to a first set of electrodes 166 and a second set of electrodes 168 via necessary wiring (not shown). The first set of electrodes 166 form a first switch S₁. The second set of electrodes 168 are enclosed by the dissolvable latch 164 (protecting the second set of electrodes 168 from contacting the gastrointestinal content, specifically the gastrointestinal fluid, in the digestive system) and are engageable with the circumferential retaining-shoulder 150 of the end piece 148 thereby forming a second switch S₂. Both switches S₁ and S₂ are in an open or off configuration when the piston 124 is retained at the rear position.

FIG. 1B is a circuit diagram of the circuit structure 118 showing the sensing circuitry 200 when the piston 124 is retained at the rear position (i.e., when the ingestible apparatus 100 is in the initial state T_{A}). As shown, the sensing circuitry 200 comprises a RFID chip 202 such as the TROVANUNIQUE^{™} ID-100A Microtransponder (TROVANUNIQUE is a trademark of TROVAN, LTD. of United Kingdom). The RFID chip 202 also comprises an antenna (not shown) for transmitting a RFID signal and is connected to a power source 204 via a resistor 206 and the first switch S₁. The sensing circuitry 200 also comprises the second switch S₂ for short-circuiting the power source 204 and the resistor 206 thereby effectively disabling the RFID chip 202.

In these embodiments, the sensing circuitry 200 is a passive RFID circuitry and the power source 204 is the antenna harnessing RF energy from an electromagnetic field generated by a RFID reader at a nearby location (outside the body of the user) and the resistor 206 represents the internal resistance of the RFID chip 202. Those skilled in the art will appreciate that in some alternative embodiments, the sensing circuitry 200 may be an active RFID circuitry and the power source 204 is a separate power-source such as a battery and/or the resistor 206 may be a separate resistor.

As described above, when the ingestible apparatus 100 is in the initial state T_{A}, the piston 124 is configured at the rear position thereby giving rise to the maximum volume of the compartment 126 for accommodating therein a health-related substance. The surface-tension force of the liquid surface 130 at the one or more substance-release ports 128 retains the substance in the compartment 126.

The first set of electrodes 166 are separated by air thereby configuring the first switch S₁ to the open of off configuration. The dissolvable latch 164 separates the electrically conductive retaining-shoulder 150 from the second set of electrodes 168 thereby configuring the second switch S₂ to the open or off configuration.

In a health-related-substance-delivery process, the ingestible apparatus 100 in the initial state T_{A} is ingested by a user into the GI tract and a RFID reader is positioned in proximity with the body of the user and paired with the RFID chip 202 for monitoring the health-related-substance-delivery process.

The enteric-targeted coating 104 protects the shell 102 until the ingestible apparatus 100 enters a predefined area such as the stomach. The enteric-targeted coating 104 is then substantively deformed thereby configuring the ingestible apparatus 100 to a second state T_{B} as shown in FIG. 2 in which the shell 102 is exposed to the gastrointestinal content (generally containing gastrointestinal fluid) of the digestive system and starts to dissolve.

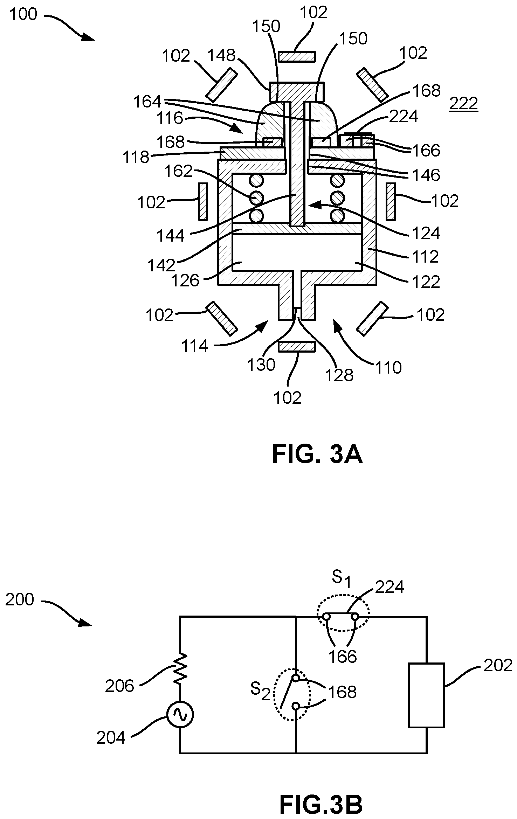

As shown in FIG. 3A, after approximately a predefined period of time, the shell 102 is substantively deformed thereby configuring the ingestible apparatus 100 to a third state Tc, in which the delivery structure 110 is exposed to the gastrointestinal content 222 and the dissolvable latch 164 starts to dissolve.

As those skilled in the art will appreciate, the gastrointestinal content 222 generally comprises gastrointestinal fluid and is conductive. Therefore, the first set of electrodes 166, when in contact with the gastrointestinal content 222, are electrically connected by the gastrointestinal content 222 (schematically represented in FIG. 3A by a thick line 224) thereby configuring the switch S₁ to a closed or on configuration. As shown in FIG. 3B, a closed circuit loop is formed from the power source 204 to the RFID chip 202 via the resistor 206 thereby triggering a signal event and enabling the RFID chip 202 to transmit a RFID signal. In the embodiments wherein the passive RFID circuitry is used, the RFID chip 202 transmits the RFID signal when a RFID reader is positioned in proximity therewith and the power source 204 is charged by the electromagnetic field of the RFID reader. In the embodiments wherein the active RFID circuitry is used, the RFID chip 202 transmits the RFID signal when the switch S₁ is configured to the closed or on configuration.

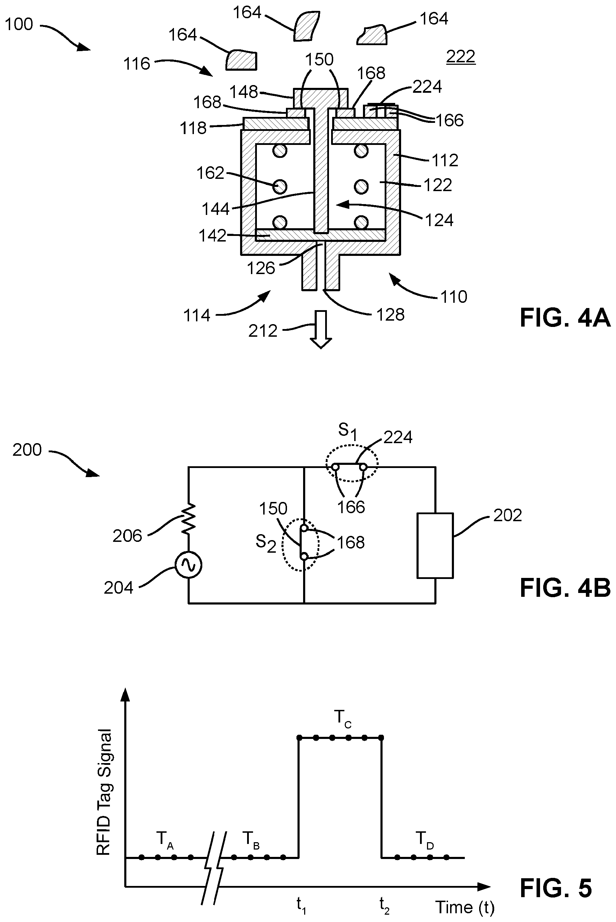

As shown in FIG. 4A, after the dissolvable latch 164 is substantively deformed, the biased spring 162 is unbiased and actuates the piston 124 to the front position thereby configuring the ingestible apparatus 100 to a fourth state T_{D}, in which the compartment 126 is configured to its minimum volume and the health-related substance therein is discharged to the target area in the GI tract through the one or more substance-release ports 128.

When the piston 124 is moved to the front position, the electrically conductive retaining-shoulder 150 engages the second set of electrodes 168 thereby configuring the second switch S₂ to the closed or on configuration. As shown in FIG. 4B, the power source 204 and the resistor 206 are short-circuited thereby triggering another signal event and disabling the RFID chip 202 to stop transmitting the RFID signal.

The time for the ingestible apparatus 100 to transit from the state Tc to the state T_{D} may be controlled by selecting the characteristics of the dissolvable latch 164 such as the dissolving rate, materials, geometry, thickness, porosity, coatings, the unbiasing force of the spring 162, and/or the like, to achieve a predefined type of drug release profile such as rapid release, extended release, and the like.

With above design, the ingestible apparatus 100 uses one or more RF signals for indicating its state. In particular, the ingestible apparatus 100 uses the absence of a RFID signal (which may be considered a first RF signal having a zero amplitude) for indicating the ingestible apparatus 100 at state T_{A}, T_{B}, or T_{D}, and uses the transmission of the RFID signal (which may be considered a second RF signal having a non-zero amplitude) for indicating the ingestible apparatus 100 at state Tc for releasing the health-related-substance.

FIG. 5 shows the output of a tracking device such as a RFID reader monitoring the ingestible apparatus 100 during a health-related-substance-delivery process wherein the dots represent the outputs of the RFID reader which form an output curve represented by the solid lines. As can be seen, the RFID reader provides a first-type output (no RFID signal detected) until time ti, and then provides a second-type output (the RFID signal detected) until time t₂ when the RFID reader turns back to the first-type output. Such an output pattern indicates that the ingestible apparatus 100 passed through the GI tract and arrived the target area approximately at time ti when the shell 102 and the coating 104 had been substantively deformed and a substance-release sub-process was started. At time t₂, the health-related substance had been discharged into the digestive system and the substance-release sub-process was completed.

Those skilled in the art will appreciate that the states T_{A} and T_{B} of the ingestible apparatus 100 may not be differentiable by the RFID reader and thus the states T_{A} and T_{B} may be considered a same (detectable) state.

In above embodiments, the one or more substance-release ports 128 are on the front wall of the housing 112. In some alternative embodiments, at least one of the one or more substance-release ports 128 may be on a sidewall of the housing 112.

In above embodiments, the enteric-targeted coating 104 provides a regional delay for protecting the shell 102 and the content therein before the ingestible apparatus 100 arrives a specific region, and the shell 102 provides a time delay before the releasing of the health-related-substance starts.

In some alternative embodiments, the ingestible apparatus 100 may not comprise an enteric-targeted coating 104. Rather, the ingestible apparatus 100 relies on the time delay provided by the shell 102 to ensure the ingestible apparatus 100 arrive the target area before the releasing of the health-related-substance starts. However, compared to the embodiments shown in FIG. 1A, the shell 102 may need to be thicker so as to provide a longer time delay. Moreover, the time delay provided by the shell 102 in these embodiments is generally selected as an average time for the ingestible apparatus 100 to travel from the mouth to the target area. However, as the actual travel time depends on the specific user's body condition and thus may be significantly different to the average travel time, the ingestible apparatus 100 in these embodiments may have a higher risk that the health-related substance therein is released outside the target area.

In above embodiments, the circuit structure 118 is coupled to the rear end of the housing 112. In some alternative embodiments, the circuit structure 118 may be coupled to the housing 112 at other suitable locations such as in the chamber 122 of the housing 112 and is electrically connected to the first and second sets of electrodes 166 and 168 via necessary wiring.

In some alternative embodiments, the RFID chip 202 of the ingestible apparatus 100 may continuously transmit RF signals during the entire health-related-substance-delivery process. In the initial state T_{A}, the switches S₁ and S₂ are configured to their open or off configuration and the RFID chip 202 transmits a first RF signal with a first set of property settings (such as amplitude, frequency, modulation, coding, and/or the like). When the shell 102 and the coating 104 are sufficiently deformed and the ingestible apparatus 100 is in the state Tc, the first switch S₁ is configured to its closed or on configuration by the gastrointestinal content which triggers the RFID chip 202 to transmit a second RF signal with a second set of property settings. When the health-related substance is released and the second switch S₂ is configured to its closed or on configuration by the electrically conductive retaining-shoulder 150, the RFID chip 202 is configured to transmit a third RF signal with a third set of property settings. In above embodiments, the third RF signal is the same as the first RF signal.

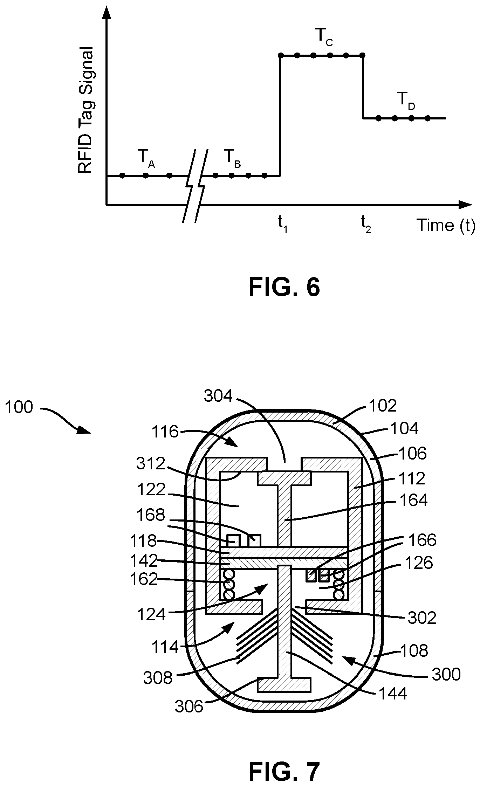

In some related embodiments, When the health-related substance is released and the second switch S₂ is configured to its closed or on configuration by the electrically conductive retaining-shoulder 150, the RFID chip 202 is configured to transmit a third RF signal different from the first RF signal (e.g., with a different amplitude), as shown in FIG. 6.

FIG. 7 shows an ingestible apparatus 100 at an initial state T_{A}, according to some embodiments of this disclosure. The ingestible apparatus 100 in these embodiments is an ingestible sampling apparatus for sampling mucosal-layer samples such as mucosal cells in the digestive system of a user.

The ingestible apparatus 100 comprises a dissolvable shell 102 coated with an enteric-targeted coating 104 and a sampling structure 300 enclosed in the dissolvable shell 102. The dissolvable shell 102 and the enteric-targeted coating 104 are similar to those described in above embodiments.

Enclosed in the shell 102, the sampling structure 300 comprises a housing 112 having an access port 302 at the front end 114 and a sample-communication port 304 at the rear end 116. The housing 112 may comprise two portions coupled together via suitable coupling means such as threading, glue, screws, an/or the like and form a chamber 122.

A piston 124 is received in the chamber 122 movable between a front position adjacent the front end 114 and a rear position adjacent the rear end 116 thereof and defines a compartment 126 between the piston 124 and the access port 302 at the front end 114 for receiving mucosal-layer samples to be collected from the digestive system.

The piston 124 comprises a piston plate 142 with a piston mandrel 144 extending forwardly from the piston plate 142 out of the housing 112 through the access port 302 to a stopper 306 having a diameter greater than that of the access port 302. The piston mandrel 144 comprises a mucosal-layer brush 308 having a plurality of bristles and at a suitable position such that the mucosal-layer brush 308 is outside the housing 112 when the ingestible sampling apparatus 100 is at the initial state T_{A}, and may be at least partially retracted into the housing after sampling.

In some embodiments, the bristles may comprise a fluid-absorbent material (i.e., a material suitable for absorbing gastrointestinal fluid) such as hydrophilic fibers, e.g., polyvinyl alcohol (PVA), to improve the collection of a mucosal sample from the mucosal layer of the GI tract in combination with the more fluid luminal sample from the intraluminal space.

In particular, fluid-absorbent material may be suitable for absorbing gastrointestinal content. When absorbing fluid upon contact with the gastrointestinal contents, the material may change the properties of the bristles such that their contact with the mucosal layer is further improved. For example, the bristles of increased stiffness may be more adept at scratching off a mucosal sample from the mucosal layer, or contact with the mucosal layer may be improved.

When the ingestible sampling apparatus 100 is in an initial state T_{A}, the piston 124 is configured at the front position adjacent the front end 114 of the chamber 122 and is retained at the front position by a dissolvable latch 164 engaging the piston plate 142 and the rear wall of the housing 112, thereby configuring the compartment 126 to its minimum volume and extending the mucosal-layer brush 308 out of the housing 112. The dissolvable latch 164 also closes the sample-communication port 304 at the rear end 116.

An actuation structure comprising a biased spring 162 engages the piston 124 for moving the piston 124 to the rear position when the dissolvable latch 164 is dissolved. In the example shown in FIG. 7, the biased spring 162 is a compressed spring located in the chamber 122 between the front end 114 and the piston 124 (i.e., in the compartment 126). The spring 162 is extendable to "push" the piston 124 towards the rear end 116 to increase the volume of the compartment 126 and retract the mucosal-layer brush 308 thereinto. In some alternative embodiments, the biased spring 162 may be an extended spring located in the chamber 122 between the rear end 116 and the piston 124. The spring 162 is retractable to "pull" the piston 124 towards the rear end 116 to increase the volume of the compartment 126 and retract the mucosal-layer brush 308 thereinto.

A circuit structure 118 is coupled to a rear surface of the piston plate 142. The circuit structure 118 comprises a sensing circuitry (not shown) electrically connected to a first set of electrodes 166 on the front surface of the piston plate 142 and a second set of electrodes 168 on the rear side of the circuit structure 118. The first set of electrodes 166 form a first switch S₁. The second set of electrodes 168 are engageable with an electrically conductive surface such as a corresponding portion 312 of the inner surface of the rear wall of the housing 112 thereby forming a second switch S₂.

When the piston 124 is retained at the front position (i.e., the ingestible sampling apparatus 100 is in the initial state T_{A}), the circuit diagram of the circuit structure 118 is similar to that shown in FIG. 1B. Both switches S₁ and S₂ are in an open or off configuration.

In a sampling process, the ingestible sampling apparatus 100 in the initial state T_{A} is ingested by a user into the GI tract and a RFID reader is positioned in proximity with the body of the user and paired with the RFID chip 202 for monitoring the sampling process.

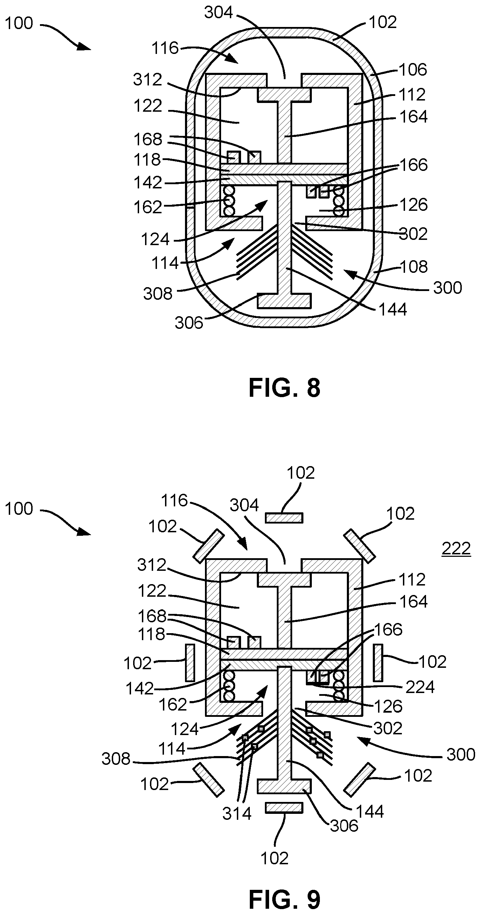

Similar to the health-related-substance-delivery process described above, the enteric-targeted coating 104 protects the shell 102 until the ingestible sampling apparatus 100 enters the stomach. The enteric-targeted coating 104 is then substantively deformed thereby configuring the ingestible sampling apparatus 100 to a second state T_{B} as shown in FIG. 8 in which the shell 102 is exposed to the gastrointestinal content of the digestive system and starts to dissolve.

As shown in FIG. 9, after approximately a predefined period of time, the shell 102 is substantively deformed thereby configuring the ingestible sampling apparatus 100 to a third state Tc, in which the sampling structure 300 is exposed to the gastrointestinal content 222 of the digestive system. While the gastrointestinal content 222 causes the dissolvable latch 164 to start dissolving, the mucosal-layer brush 308 is exposed to the digestive system and interacts with the mucosal layer thereof to collect gastrointestinal samples 314.

Herein, the gastrointestinal samples may comprise all substances contained in or being part of the GI tract of the user. This may include but not limit to, tissue, cells, including those from the mucosal layer, gastrointestinal fluid, microbiome, viruses, biological markers as well as foreign substances introduced to the GI tract of the user. For example, pharmaceutical agents or markers, probiotics or other treatments administered to the user, chemicals ingested by or having been exposed to the user. The gastrointestinal samples may be collected from the intra-luminal space, which substantively include flowing gastrointestinal fluid and may also include some cells (e.g., debris from elsewhere in the user's body or free-floating cells that have detached from the mucosal layer). Samples collected from the intra-luminal space are referenced as luminal samples. The gastrointestinal samples can also be collected from the mucosal layer where there may be an increased density of mucosal cells adhering to the lining of the GI tract. These are more difficult to obtain, and are herein referred to as mucosal-layer samples. Herein, the terms "gastrointestinal samples" and "gastrointestinal-content samples" may be used interchangeably, which may comprise at least one of the luminal samples and mucosal-layer samples.

The gastrointestinal content 222 enters the chamber 122 from the access port 302. The electrically conductive gastrointestinal content 222 connects the first set of electrodes 166 (schematically represented in FIG. 9 by a thick line 224) thereby configuring the switch S₁ to a closed or on configuration. A closed circuit loop is formed thereby enabling the RFID chip 202 to transmit a RFID signal. The circuit diagram of the circuit structure 118 is similar to that shown in FIG. 3B.

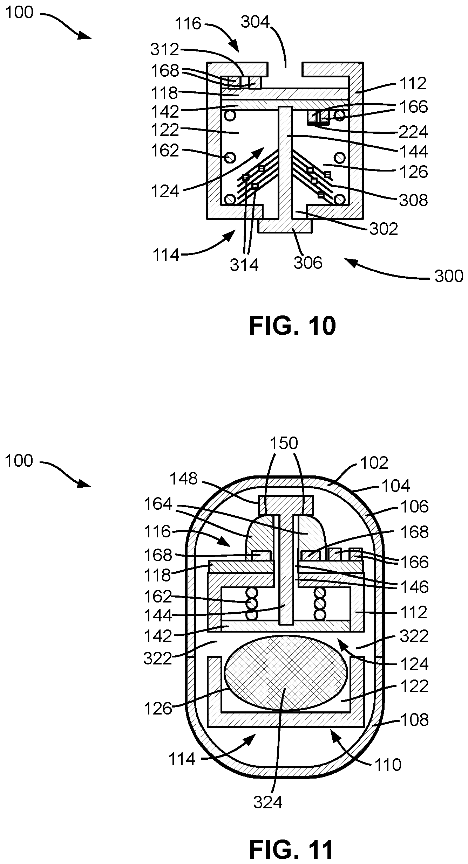

As shown in FIG. 10, after the dissolvable latch 164 is substantively deformed, the biased spring 162 is unbiased and actuates the piston 124 to the rear position thereby configuring the ingestible sampling apparatus 100 to a fourth state T_{D}, in which the compartment 126 is configured to its maximum volume. The mucosal-layer brush 308 and the collected mucosal-layer samples 314 are retracted into the compartment 126. The stopper 306 engages the housing 112 to close the access port 302 thereby preventing content-exchange between the compartment 126 and the digestive system. The captured mucosal-layer samples 314 are thus isolated from the digestive system and are prevented from being contaminated when the ingestible sampling apparatus 100 continues its travel in the GI tract and eventually discharged therefrom.

When the piston 124 is moved to the rear position, the second set of electrodes 168 engage the electrically conductive portion 312 of the rear wall of the housing 112 thereby configuring the second switch S₂ to the closed or on configuration. The power source 204 and the resistor 206 are short-circuited thereby disabling the RFID chip 202 to stop transmitting the RFID signal. The circuit diagram of the circuit structure 118 is similar to that shown in FIG. 4B. The RFID signal transmission is similar to that described above, and the output of the RFID reader is similar to that shown in FIG. 5.

In some embodiments, the ingestible sampling apparatus 100 shown in FIG. 7 may be used for collecting the luminal samples from the intraluminal space. In these embodiments, the ingestible sampling apparatus 100 may not comprise a mucosal-layer brush 308.

FIG. 11 shows an ingestible apparatus 100 at an initial state T_{A}, according to some embodiments of this disclosure. The ingestible apparatus 100 in these embodiments is an ingestible sampling apparatus for fluid sampling in the digestive system of a user. The ingestible sampling apparatus 100 in these embodiments is similar to the ingestible apparatus shown in FIG. 1A with differences in the housing 112 and the piston 124. Therefore, the description of some common structural features is omitted.

In these embodiments, the housing 112 forms a chamber 122 and comprises one or more sample-communication ports 322 on a sidewall thereof at a distance to the front wall. Thus, the portion of the chamber 122 between the front wall of the housing 112 and the one or more sample-communication ports 322 forms a compartment 126 which receives therein a suitable fluid-absorbent material 324 such as a bundle of hydrophilic fibers for retaining the luminal samples therein.

The piston 124 is similar to that shown in FIG. 1A. However, in these embodiments, the piston 124 is retained at a position rearward to the one or more sample-communication ports 322 such that, during gastrointestinal-content sampling, luminal samples entering the one or more sample-communication ports 322 may flow into the compartment 126 and accumulate therein.

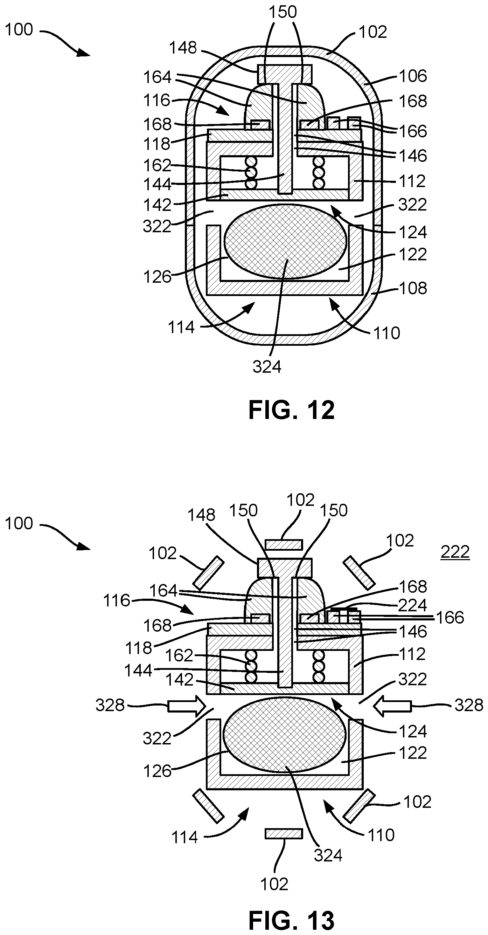

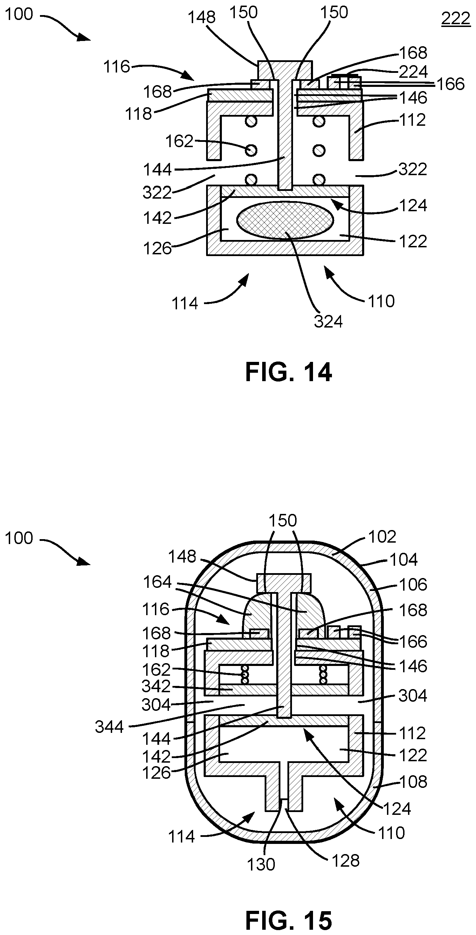

The luminal-sampling process is shown in FIGs. 11 to 14 which is similar to the health-related-substance-delivery process shown in FIGs. 1A, 2A, 3A, and 4A except the following differences:
(i) At state Tc (see FIG. 13), the dissolvable shell 102 is sufficiently deformed and the luminal sample (not shown) flows into the compartment 126 via the one or more sample-communication ports 322 as indicated by the arrows 328 due to gravity and the peristaltic force of the digestive system. The fluid-absorbent material 324 facilitates the accumulation of the luminal samples in the compartment 126.
(ii) At state T_{D} (see FIG. 14), the dissolvable latch 164 is substantively deformed and the biased spring 162 actuates the piston 124 to the front position forward to the one or more sample-communication ports 322 which effectively isolates the compartment 126 from the one or more sample-communication ports 322 thereby closing the compartment 126 and sealing the collected luminal samples therein.

The circuit diagrams in the states T_{A}, T_{B}, Tc, and T_{D} are similar to those shown in FIGs. 1B, 2B, 3B, and 4B, and the tracking process similar to that described above with the RFID reader outputting two types of outputs as shown in FIG. 5.

FIG. 15 shows an ingestible apparatus 100 at an initial state T_{A}, according to some embodiments of this disclosure. The ingestible apparatus 100 in these embodiments is an ingestible health-related-substance-delivery and gastrointestinal-content-sampling apparatus for delivering a health-related substance and for sampling gastrointestinal/luminal content in the digestive system of a user.

The ingestible apparatus 100 in these embodiments comprises a dissolvable shell 102 coated with an enteric-targeted coating 104 similar to those shown in FIG. 1A.

Enclosed in the dissolvable shell 102, the ingestible apparatus 100 comprises a delivery-and-sampling structure 110 which comprises a housing 112 having a front end 114 and a rear end 116 and a circuit structure 118 coupled to the rear end 116 of the housing 112. The circuit structure 118 and the first and second sets of electrodes 166 and 168 are similar to those shown in FIG. 1A.

The housing 112 may comprise two portions coupled together via suitable coupling means such as threading, glue, screws, an/or the like to form a chamber 122. The housing 112 comprises a set of one or more substance-release ports 128 adjacent the front end 114 thereof and a set of one or more sample-communication ports 304 at a rear side of the substance-release ports 128 and spaced therefrom.

A piston 124 is movably received in the chamber 122 and is secured at a rear position adjacent the rear end 116 thereof by a dissolvable latch 164 in a same manner as shown in FIG. 1A. The piston 124 engages an actuation structure comprising a biased spring 162. When the dissolvable latch 164 is substantively deformed, the biased spring 162 may be unbiased and actuate the piston 124 to move from the rear position to a front position adjacent the front end 114.

The piston 124 comprises a front piston plate 142 and a rear piston plate 342 respectively located on the front and rear sides of the sample-communication ports 304 thereby defining a first compartment 126 between the front piston plate 142 and the front end 114 for accommodating therein one or more active compounds in a liquid form and a second compartment 344 between the front and rear piston plates 142 and 342 for receiving therein the gastrointestinal-content samples to be collected. A piston mandrel 144 couples the front and rear piston plates 142 and 342 to an end piece 148 outside the rear end of the housing 112. Similar to that described above, the end piece 148 forms an electrically conductive, circumferential retaining-shoulder 150 engageable with the second set of electrodes 168.

At state T_{A}, the first compartment 126 is in communication with the set of substance-release ports 128. Each substance-release port 128 has a small dimension allowing the fluid in the compartment 126 to form a liquid surface 130 at the substance-release port 128 with a surface-tension force and/or the friction of the sidewall of the substance-release port 128 that are sufficient for retaining the fluid in the compartment 126. The second compartment 344 is in communication with the set of sample-communication ports 304.

The ingestible apparatus 100 may be used for collecting gastrointestinal samples in a target area in the GI tract of a user and then releasing a health-related substance thereto.

In a health-related-substance-delivery and gastrointestinal-sampling process, the ingestible apparatus 100 configured in the state T_{A} is ingested into the GI tract. While travelling in the GI tract, the ingestible apparatus 100 transits to states T_{B} and Tc as described above.

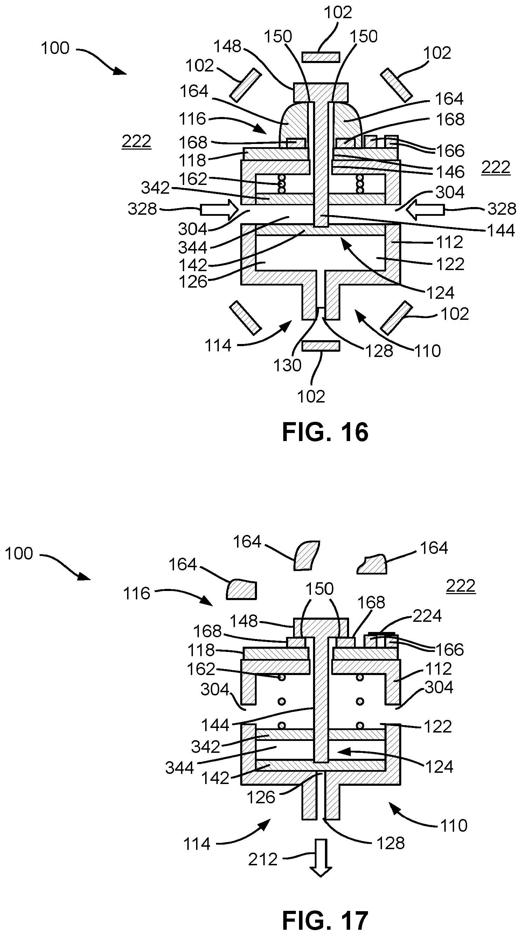

FIG. 16 shows the ingestible apparatus 100 in the state Tc. As shown, the shell 102 and coating 104 (not shown) are substantively deformed and the gastrointestinal sample 222 flows into the second compartment 344 through the set of sample-communication ports 304 as indicated by the arrows 328. In this state, the health-related substance is substantively retained in the first compartment 126.

When the dissolvable latch 164 is dissolved, the ingestible apparatus 100 transits to the state T_{D}. As shown in FIG. 17, the biased spring 162 is unbiased and actuates the piston 124 to move to the front position at which the front piston plate 142 discharges the health-related substance from the first compartment 126 out of the housing 112 into the target area of the GI tract, as indicated by the arrow 212. The rear piston plate 342 is moved to the front side of the set of sample-communication ports 304 thereby isolating the second compartment 344 from the set of sample-communication ports 304 and sealing the collected gastrointestinal samples in the second compartment 344.

The circuit diagrams in the states T_{A}, T_{B}, Tc, and T_{D} are similar to those shown in FIGs. 1B, 2B, 3B, and 4B, and the tracking process is similar to that described above with the RFID reader outputting two types of outputs as shown in FIG. 5.

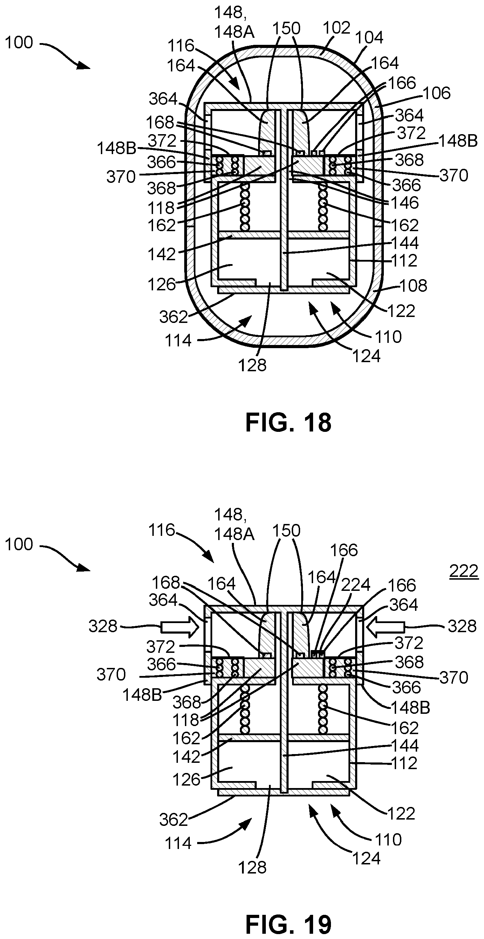

FIG. 18 shows an ingestible apparatus 100 at an initial state T_{A}, according to some embodiments of this disclosure. The ingestible apparatus 100 in these embodiments is an ingestible health-related-substance-delivery and gastrointestinal-content-sampling apparatus for delivering a health-related substance having one or more active compounds and for sampling gastrointestinal content in the digestive system of a user.

The ingestible apparatus 100 in these embodiments comprises a dissolvable shell 102 coated with an enteric-targeted coating 104 similar to those shown in FIG. 1A.

Enclosed in the dissolvable shell 102, the ingestible apparatus 100 comprises a delivery-and-sampling structure 110 which comprises a housing 112 having a front end 114 and a rear end 116 and a circuit structure 118 coupled to the rear end 116 of the housing 112. The circuit structure 118 and the first and second sets of electrodes 166 and 168 are similar to those shown in FIG. 1A.

The housing 112 comprises a chamber 122 and a set of one or more substance-release and gastrointestinal-content-sampling ports 128 adjacent the front end 114 thereof. A piston 124 is movably received in the chamber 122 and is secured at a rear position adjacent the rear end 116 thereof by a dissolvable latch 164 in a same manner as shown in FIG. 1A. The piston 124 engages a substance-delivery actuation structure comprising a biased (e.g., compressed) substance-delivery spring 162. When the dissolvable latch 164 is substantively deformed, the biased substance-delivery spring 162 may be unbiased and actuate the piston 124 to move from the rear position to a front position adjacent the front end 114.

The piston 124 comprises a sealable-component 362 about a front end thereof and engaging an exterior surface of the housing 112 about the set of substance-release and gastrointestinal-content-sampling ports 128 and a piston plate 142 at a distance to the front end 144 of the housing 112 to form a compartment 126 for accommodating therein one or more health-related-substances in a liquid or a semi-liquid form and also for receiving therein the gastrointestinal samples to be collected. A piston mandrel 144 couples the sealable-component 362 and the piston plate 142 to an end cap 148 outside the rear end of the housing 112.

In these embodiments, the end cap 148 is in the form of a cylindrical sleeve having a rear wall 148A and a sidewall 148B and slidably receiving therein a rear portion of the housing 112. Similar to that described above, the rear wall 148A of the end cap 148 comprises an electrically conductive, circumferential retaining-shoulder 150 engageable with the second set of electrodes 168. One or more windows 364 are distributed on the sidewall 148B and/or the rear wall 148A for establishing communication between the interior of the end cap 148 and the digestive system when the shell 102 and coating 104 are substantively deformed.

The delivery-and-sampling structure 110 further comprises a sampling actuation-structure comprising a biased (e.g., compressed) outer sampling-spring 366 and a biased (e.g., compressed) inner sampling-spring 368 coupled to the rear end of the housing 112 and received in the end cap 148. Both sampling-springs 366 and 368 are retained in their biased configurations by a dissolvable circular block 370. A rupture disk 372 such as an undissolvable film is coupled to a rear end of the dissolvable block 370 for protecting the dissolvable block 370 and the sampling-springs 366 and 368 retained therein from contacting gastrointestinal content.

The ingestible apparatus 100 may be used for delivering a health-related substance to a target area in the GI tract of a user and then collecting gastrointestinal samples therein after a time delay from the health-related-substance-delivery.

In a substance-delivery and gastrointestinal-sampling process, the ingestible apparatus 100 configured in the state T_{A} is ingested into the GI tract. While travelling in the GI tract, the ingestible apparatus 100 transits to states T_{B} and Tc as described above.

FIG. 19 shows the ingestible apparatus 100 in the state Tc. As shown, the shell 102 and coating 104 (not shown) are substantively deformed and the gastrointestinal samples 222 flow into the interior of the end cap 148 via the windows 364 as indicated by the arrows 328. The dissolvable latch 164 then starts to dissolve.

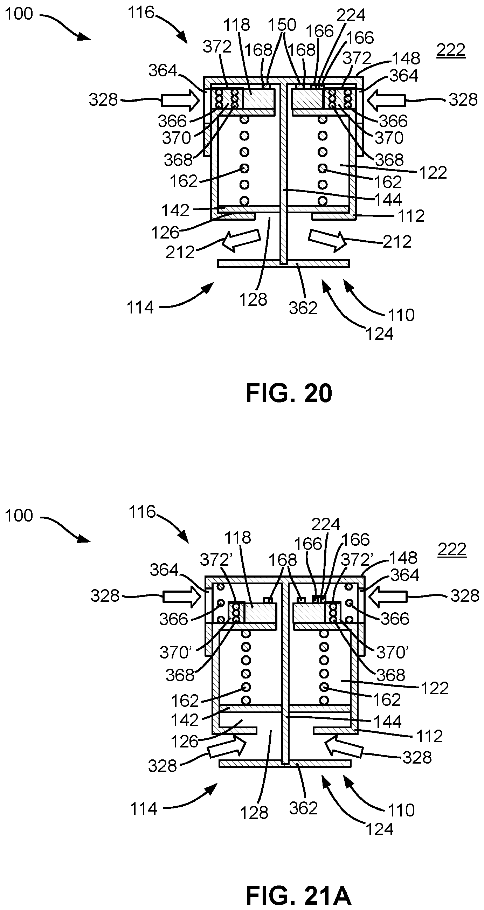

When the dissolvable latch 164 is substantively deformed, the ingestible apparatus 100 transits to the state T_{D}. As shown in FIG. 20, the biased substance-delivery spring 162 is unbiased and actuates the piston 124 to move to the front position at which the piston plate 142 discharges the health-related substance from the compartment 126 out of the housing 112 into the target area of the GI tract, as indicated by the arrow 212. The windows 364 of the end cap 148 are aligned with the dissolvable circular block 370 and introduce gastrointestinal content thereto. The dissolvable circular block 370 then starts to dissolve.

The circuit diagrams in the states T_{A}, T_{B}, Tc, and T_{D} are similar to those shown in FIGs. 1B, 2B, 3B, and 4B, and the tracking process is similar to that described above with the RFID reader outputting two types of outputs as shown in FIG. 5.

When the portion of the dissolvable circular block 370 retaining the outer sampling-spring 366 is substantively deformed, the ingestible apparatus 100 transits to a state T_{E}. As shown in FIG. 21A, the biased outer sampling-spring 366 is unbiased, breaks the rupture disk 372, and overcomes the resistance of the substance-delivery spring 162 to rearwardly actuate the end cap 148. Consequently, the piston 124 is moved rearwardly to an intermediate position increasing the volume of the compartment 126. Gastrointestinal samples 222 then flow through the gap between the sealable-component 362 and the front end of the housing 112 and through the substance-release and gastrointestinal-content-sampling ports 128 into the compartment 126.

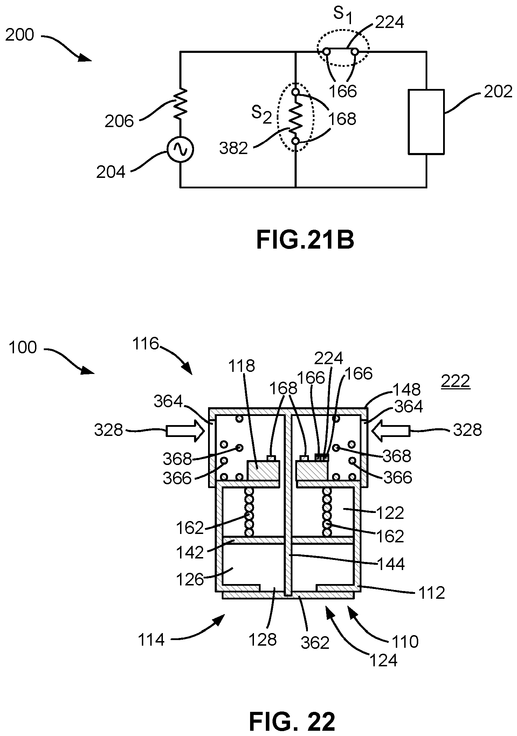

The circuit diagram of the ingestible apparatus 100 in the state T_{E} is shown in FIG. 21B. As the electrically conductive retaining-shoulder 150 is not in contact with the second set of electrodes 168 and as the second set of electrodes 168 are spaced at a relatively large distance, switch S₂ may be considered a resistor 382 with a relatively large resistance. The RFID chip 202 may be configured to transmit another RFID signal with a different set of property settings.

In the state T_{E}, the remaining portion 370' of the dissolvable circular block 370 continues to dissolve.

When the dissolvable circular block portion 370' retaining the inner sampling-spring 368 is substantively deformed, the ingestible apparatus 100 transits to a state T_{F}. As shown in FIG. 22, the biased inner sampling-spring 368 is unbiased, breaks the remaining rupture disk portion 372', and overcomes the resistance of the substance-delivery spring 162 to further rearwardly actuate the end cap 148. Consequently, the piston 124 is moved rearwardly to the rear position giving rise to the maximum volume of the compartment 126. The sealable-component 362 engages the exterior surface of the housing 112 about the substance-release and gastrointestinal-content-sampling ports 128 thereby closing the substance-release and gastrointestinal-content-sampling ports 128 and sealing the collected gastrointestinal samples in the compartment 126.

The circuit diagram of the ingestible apparatus 100 in the state T_{F} is similar to that shown in FIG. 21B.

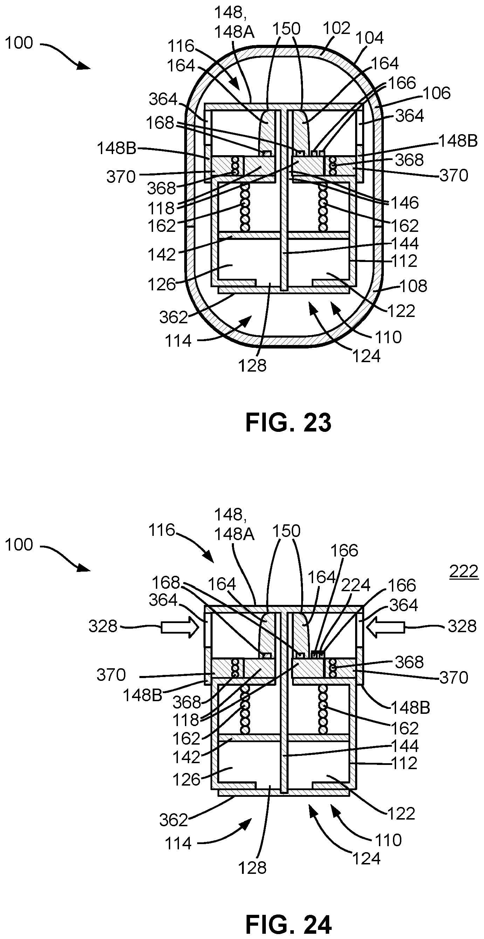

In the embodiments shown in FIGs. 18 to 22, the ingestible apparatus 100 comprises two sampling-springs 366 and 368. In some embodiments as shown in FIG. 23, the ingestible apparatus 100 may only comprise one sampling-spring 368.

In these embodiments, the dissolvable circular block 370 comprises a suitable dissolvable material that may gradually dissolve without abrupt deformation. Moreover, the ingestible apparatus 100 does not comprise any rupture disk for isolating the dissolvable block 370 from the gastrointestinal content.

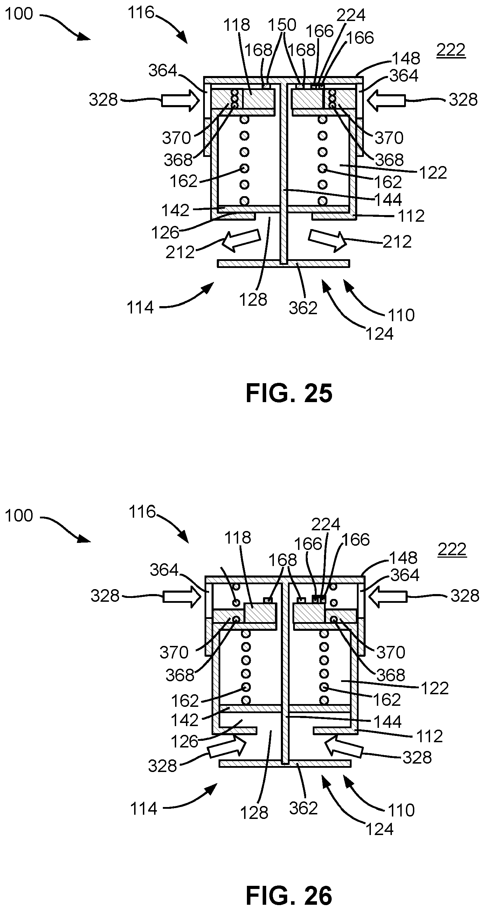

As shown in FIGs. 23 to 27, when the ingestible apparatus 100 is ingested into the GI tract, and after the shell 102 and coating 104 are substantively deformed, the dissolvable latch 164 is in contact with gastrointestinal content and starts to dissolve (FIG. 24). When the dissolvable latch 164 is substantively deformed, the substance-delivery spring 162 actuates the piston 144 to the front position thereby discharging the health-related substance from the compartment 126 into the target area of the GI tract (FIG. 25). Meanwhile, the dissolvable block 370 is in contact with the gastrointestinal content and starts to dissolve.

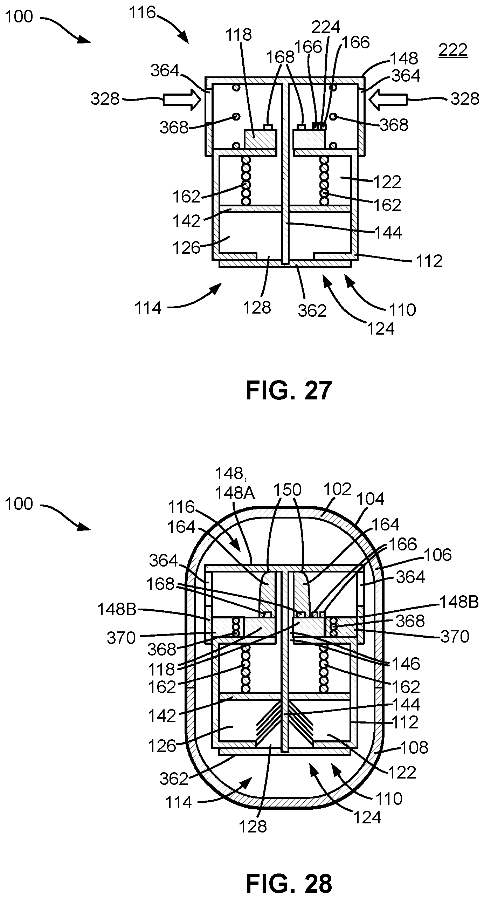

With the gradual dissolution of the dissolvable block 370, the sampling-spring 368 gradually actuates the piston 144 to move towards the rear position thereby increasing the volume of the compartment 126 and allowing the gastrointestinal content to flow thereinto (FIG. 26). When the dissolvable block 370 is substantively dissolved, the sampling-spring 368 actuates the piston 144 to the rear position thereby closing the port 128 to seal the collected gastrointestinal samples in the compartment 126 (FIG. 27).

The states and circuit diagrams of the ingestible apparatus 100 in these embodiments are similar to those described in the embodiments shown in FIGs. 18 to 22.

FIG. 28 shows an ingestible apparatus 100, according to some embodiments of this disclosure. The ingestible apparatus 100 in these embodiments is similar to that shown in FIGs. 23 to 27. However, in these embodiments, the ingestible apparatus 100 comprises a mucosal-layer brush 308 attached to the piston mandrel 144 about a distal end thereof. At the initial state T_{A} as shown in FIG. 28, the mucosal-layer brush 308 is received in the compartment 126 with the health-related substance to be delivered.

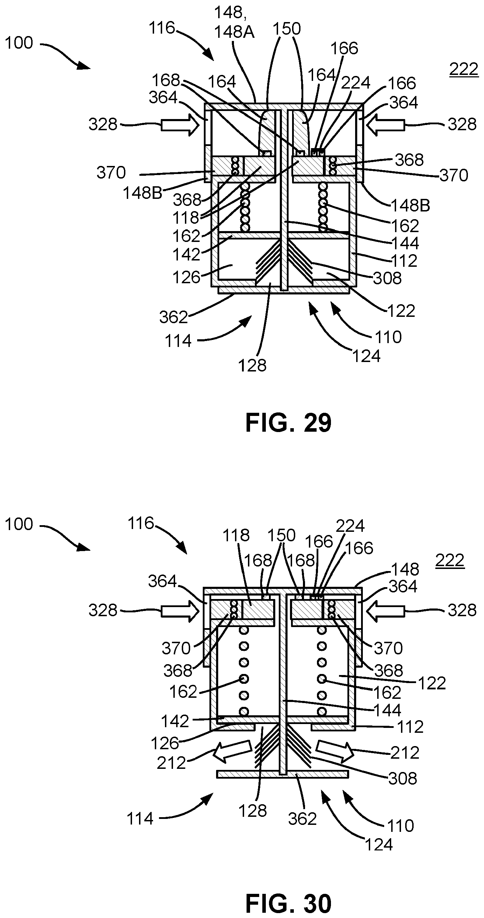

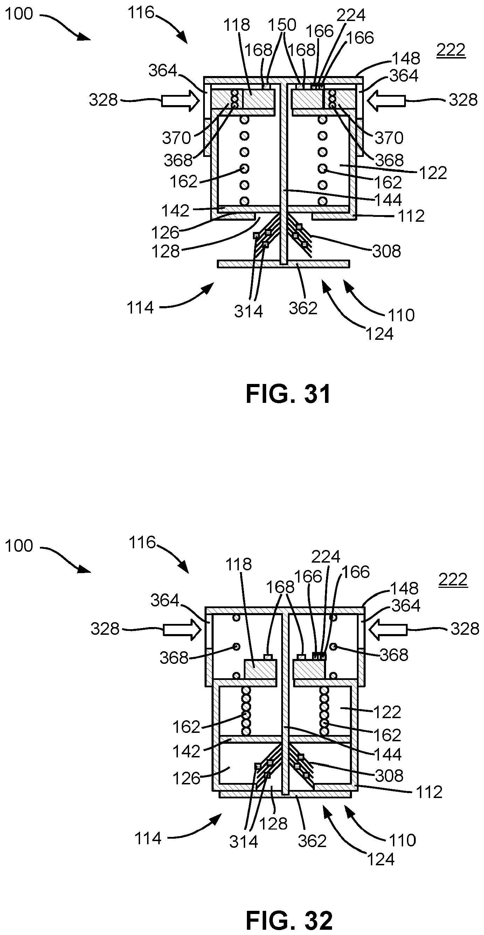

The substance-delivery and luminal-sampling process is shown in FIGs. 28 to 32. When the ingestible apparatus 100 is ingested into the GI tract, and after the shell 102 and coating 104 are substantively deformed, the gastrointestinal content 222 flows into the interior of the end cap 148 via the windows 364 as indicated by the arrows 328. The dissolvable latch 164 is then in contact with gastrointestinal content and starts to dissolve (FIG. 29). When the dissolvable latch 164 is substantively deformed, the substance-delivery spring 162 actuates the piston 144 to the front position thereby discharging the health-related substance from the compartment 126 into the target area of the GI tract (FIG. 30). The mucosal-layer brush 308 is extended out of the compartment 126 for collecting mucosal-layer samples. The dissolvable block 370 is in contact with the gastrointestinal content and starts to dissolve (FIG. 31).

When the dissolvable block 370 is substantively deformed, the sampling-spring 368 actuates the piston 144 to the rear position thereby retracting the mucosal-layer brush 308 and the collected mucosal-layer samples into the compartment 126 and closing the port 128 to seal the compartment 126 (FIG. 32).

In these embodiments, the dissolvable block 370 may not need to be gradually dissolved. Rather, it may be abruptly deformed after a predefined period of time to retract the mucosal-layer brush 308 and the collected mucosal-layer samples into the compartment 126 and close the port 128 to seal the compartment 126.

The states and circuit diagrams of the ingestible apparatus 100 in these embodiments are similar to those described in the embodiments shown in FIGs. 18 to 22.

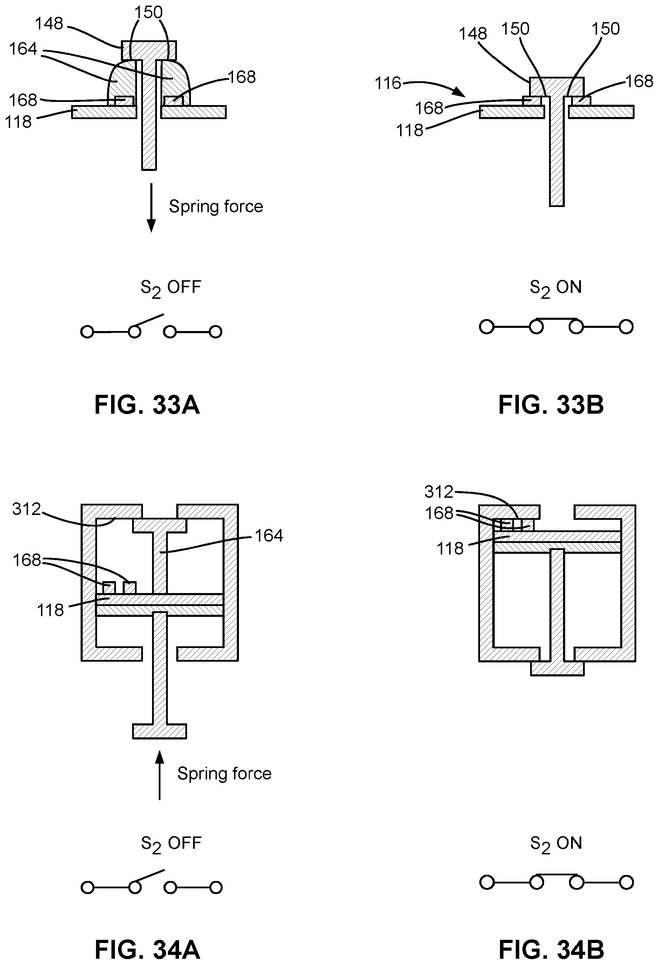

In above embodiments, the second switch S₂ is implemented by using a second set of electrodes 168 engageable with an electrically conductive surface such as the electrically conductive retaining-shoulder 150. The second switch S₂ may be turned from off (also see FIG. 33A, the second switch S₂ off) to on (also see FIG. 33B, the second switch S₂ on) by a spring force.

The second switch S₂ may also be implemented by using a second set of electrodes 168 engageable with an electrically conductive portion of the rear wall of the housing 112. The second switch S₂ may be turned from off (also see FIG. 34A, the second switch S₂ off) to on (also see FIG. 34B, the second switch S₂ on) by a spring force.

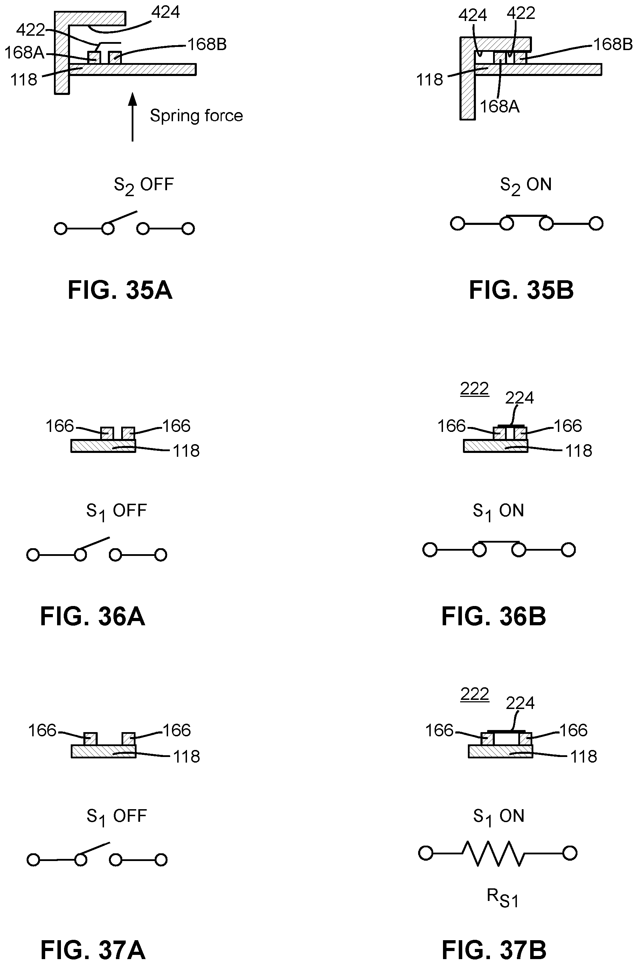

In various embodiments, the second switch S₂ may be implemented in any suitable manner. For example, as shown in FIG. 35A the second switch S₂ may comprise a set of electrodes 168A and 168B. The electrode 168A comprises an electrically conductive tab 422 extending therefrom to a location in proximity with the electrode 168B thereby configuring the second switch S₂ to an open or off configuration.

A spring force may be used to actuate the second set of electrodes 168A and 168B (in this example the circuit structure 118 and the second set of electrodes 168A and 168B thereon) towards a stopper 424 which may be a wall of the housing 112 or the end piece 148 of the piston 124, to push the electrically conductive tab 422 in contact with the electrode 168B thereby configuring the second switch S₂ to a closed or on configuration as shown in FIG. 35B.

Alternatively, a spring force may actuate the stopper 424 towards the second set of electrodes 168A and 168B to push the electrically conductive tab 422 in contact with the electrode 168B thereby configuring the second switch S₂ to a closed or on configuration.

In above embodiments, the first switch S₁ is implemented by using a first set of electrodes 166 initially electrically isolated by air and configured to an open or off configuration (also see FIG. 36A, the first switch S₁ off).

The first switch S₁ may be configured to a closed or on configuration when the first set of electrodes 166 are in contact with electrically conductive gastrointestinal-content 222. As shown in FIG. 36B, in some embodiments wherein the first set of electrodes 166 are at a short distance from each other, the resistance of the gastrointestinal content 224 therebetween is small and may be ignored. Therefore, the first switch S₁ in the closed or on configuration may be modelled as a connection wiring with zero resistance.

In some embodiments wherein the first set of electrodes 166 may be at a relatively large distance from each other (see FIG. 37A). The first switch S₁ may be configured to a closed or on configuration when the first set of electrodes 166 are in contact with electrically conductive gastrointestinal-content 222. As shown in FIG. 37B, the resistance of the gastrointestinal content 224 therebetween in these embodiments may not be ignored. Therefore, the first switch S₁ in the closed or on configuration may be modelled a resistor Rsi.

In above embodiments, the switches S₁ and S₂ are used for determining the RF signal to be transmitted for indicating the state of the ingestible apparatus 100. The first switch S₁ acts as a detection circuit for detecting gastrointestinal content. The second switch S₂ acts as a detection-circuit for detecting a volume-change of the first compartment 126 by detecting the position or movement of the piston 124. By detecting the gastrointestinal content and the volume-change of the first compartment 126, the state of the ingestible apparatus 100 may be determined which may be sent to a tracking device via one or more RF signals.

In some alternative embodiments, other gastrointestinal-content detection-circuit may be used.

In some alternative embodiments, other volume-change or piston-position detection-circuit may be used. For example, in one embodiment, the piston 124 may comprise a magnetic component such as a permanent magnet and the housing 112 may comprise a Hall-effect sensor in proximity with the magnetic component for detecting the piston position (and subsequently the volume-change of the first compartment 126). The output of the Hall-effect sensor may be used for determining the RF signal (or more specifically the property settings thereof) for indicating the state of the ingestible apparatus 100.

In another embodiment, the piston 124 may be coupled to the slider of a sliding rheostat for adjusting the resistance of the sliding rheostat for detecting the piston position which may be used for determining the RF signal (or more specifically the property settings thereof) for indicating the state of the ingestible apparatus 100.

In these embodiments, by using the sliding rheostat, the spring movement may be continuously monitored and the RF signal may be used for continuously reporting the monitored spring movement.

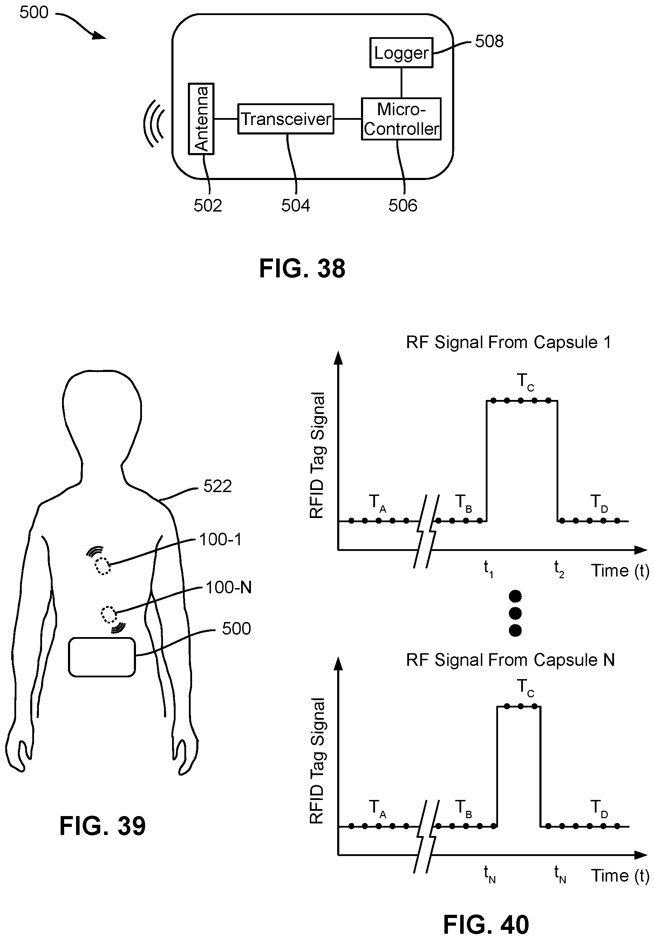

In above embodiments, a RFID reader is used as a tracking device for tracking the ingestible apparatus 100 during its travel in the GI tract. FIG. 38 is a block diagram of a wearable RFID reader 500. As shown. The wearable RFID reader 500 comprises an antenna 502, a RF-signal transceiver 504, a microcontroller 506 (which is a controlling circuitry), and a data logger 508. The microcontroller 506 is configured to command the RF-signal transceiver 504 to periodically (e.g., every five (5) minutes) interrogate the RFID chips 202 of one or more ingestible apparatus 100 using an RF signal.

As those skilled in the art will appreciate, each RFID chip 202 may transmit a RFID signal having a unique identity (ID). Therefore, a user may ingest one or more ingestible apparatuses 100 for health-related-substance-delivery, and/or gastrointestinal-content sampling as needed from either the intra-luminal space or the mucosal lining.

As shown in FIG. 39, a user 522 may digest N above-described ingestible apparatuses 100-1 to 100-N which may have different settings such as different regional dissolution characteristics, different time delays, and/or the like. Each ingestible apparatus 100 has a unique ID encoded in the RFID signal transmittable from the RFID chip 202 thereof. A wearable RFID reader 500 is attached to the user for tracking the ingestible apparatuses 100-1 to 100-N as described above. The signals detected by the wearable RFID reader 500 is shown in FIG. 40.

The microcontroller 506 of the wearable RFID reader 500 records the results of the interrogating-and-reading events of the ingestible apparatuses 100-1 to 100-N in the data logger 508.

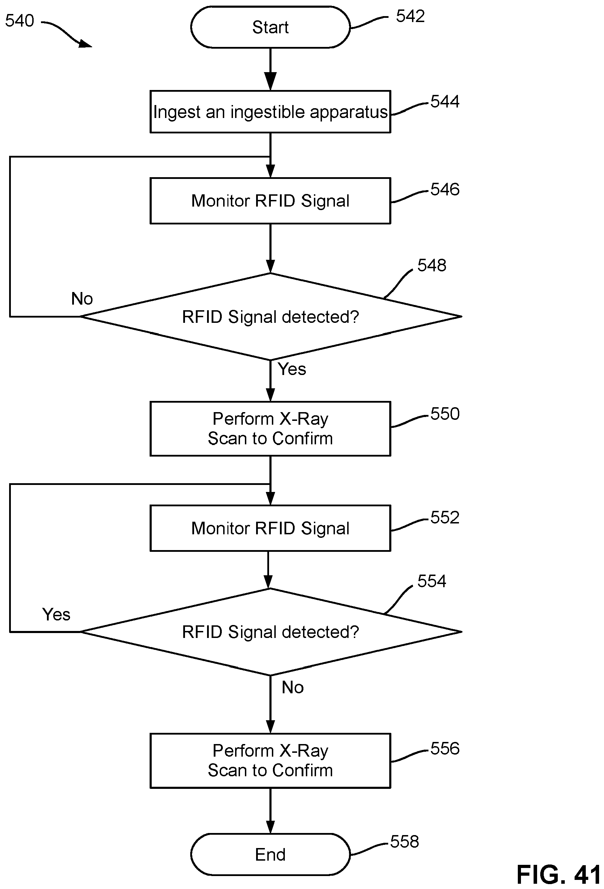

FIG. 41 is a flowchart showing the steps of a monitoring process 540 for monitoring a sampling and/or health-related-substance-delivery process using above-described ingestible apparatus 100 with X-Ray confirmation.

When the monitoring process 540 starts (step 542), a user ingests an ingestible apparatus 100 and a wearable RFID reader 500 is attached to the user for tracking the ingestible apparatus 100 (step 544). The wearable RFID reader 500 monitors the RFID signal (step 546). If no RFID signal is detected (step 548), the process loops back to step 546 and continues the monitoring of the RFID signal.

If the RFID signal is detected at step 548, a X-Ray scan is performed to confirm the detection of the ingestible apparatus 100 and to confirm that the ingestible apparatus 100 has arrived the target area (step 550). Then, the wearable RFID reader 500 continues the monitoring of the RFID signal (step 552). If the RFID signal continues to be detected (step 554), the ingestible apparatus 100 is still in the process of discharging the health-related substance and/or collecting samples of gastrointestinal content from either the intra-luminal space or the mucosal lining. The process then loops back to step 552 and continues the monitoring of the RFID signal.

If no RFID signal is detected at step 554, another X-Ray scan is performed to confirm that the ingestible apparatus 100 has completed sampling and/or health-related-substance-delivery (step 556). The process 540 then ends (step 558).

Those skilled in the art will appreciate that the X-Ray-scan steps 550 and 556 may be optional. Thus, in some alternative embodiments, the process 540 may omits at least one of steps 550 and 556.

Although in above embodiments, a RFID chip 202 and a RFID reader are used for tracking the ingestible apparatus 100, in various embodiments, the ingestible apparatus 100 may comprise any suitable RF-signal transmitter and a corresponding RF-signal receiver may be used for tracking the ingestible apparatus 100.

In some alternative embodiments, the ingestible apparatus 100 described above may not comprise any circuit structure 118. In these embodiments, no RFID reader is used for monitoring the ingestible apparatus 100 travelling in the GI tract.

In above embodiments, the ingestible apparatus 100 comprises one or more springs 162, 366, and 368 as one or more actuation structures for actuating the piston 124 to the front and/or rear positions. In some alternative embodiments, at least one of the one or more springs 162, 366, and 368 may be substituted with other suitable actuation structures such as a suitable shape-memory alloy or other memory materials, a material expandable when in contact with the gastrointestinal content, and/or the like. Depending on the actuation structure, the dissolvable block 370 may not be required.

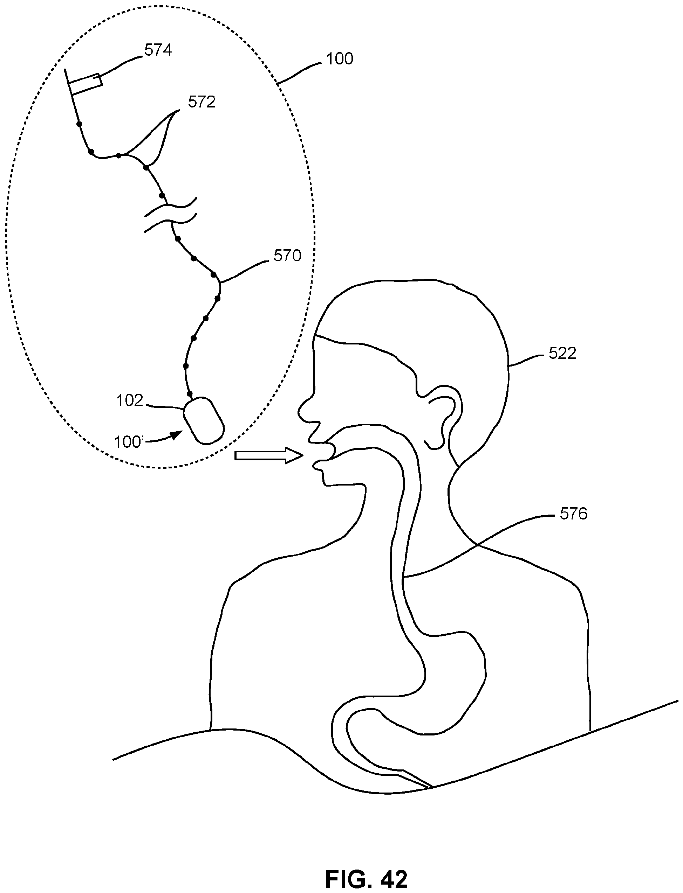

FIG. 42 shows an ingestible assembly 100 according to some alternative embodiments of this disclosure. The ingestible assembly 100 in these embodiments may be the ingestible apparatus (identified using reference numeral 100' in FIG. 42) described in above embodiments and further comprises a string 570 coupled to the shell 102. The string 570 is made of a suitable material and comprises a plurality of marks 572 such as color marks or knots distributed thereon for indicating the distance from the shell 102. The string 570 may also have a handle 574 at the opposite end thereof.

In a sampling and/or health-related-substance-delivery process, the ingestible apparatus 100' is ingested by a user 522 and goes through the GI tract 576 as described above. With the movement of the ingestible apparatus 100' in the GI tract 576, the string 570 is extended into the mouth of the user 522. By monitoring the marks of the string 570 remaining outside the user's mouth, the location of the ingestible apparatus 100' in the GI tract 576 may be monitored.

When the shell 102 of the ingestible apparatus 100' is dissolved, the string 570 is separated from the delivery structure 110 and may be pulled out from the user's mouth. Thus in these embodiments, the string 570 may be made of an undissolvable material.

In some embodiments, the string 570 may be made of a dissolvable material and may be coupled to the shell 102 or the housing 112. When the shell 102 of the ingestible apparatus 100' is dissolved, the string 570 may be left in the user's GI tract and dissolve. In these embodiments, a portion of the string 570 adjacent the handle 574 may be made of an undissolvable material.

In some embodiments wherein the ingestible apparatus 100 is configured for sampling, the ingestible apparatus 100 may be used for collecting other mucosal-layer samples than mucosal-layer samples.

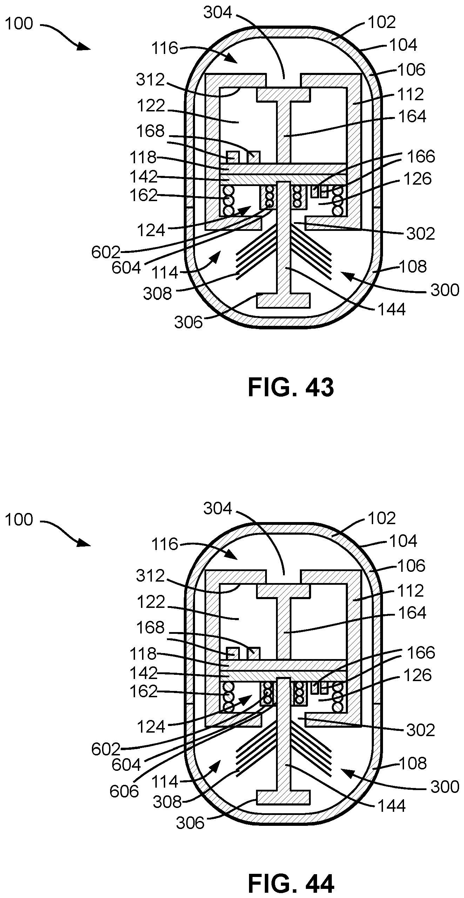

FIG. 43 shows an ingestible apparatus 100 in some embodiments. The ingestible apparatus 100 in these embodiments is similar to that shown in FIG. 7 except that the piston mandrel 144 is rotatable about a longitudinal axis thereof. The piston mandrel 144 is coupled to a torsion structure such as a twisted torsion-spring 602 retained in a dissolvable block 604 such that the torsion structure would not release the energy stored therein before the dissolvable block 604 is substantively deformed.

When the ingestible apparatus 100 is ingested in the GI tract and the shell 102 and coating 104 are substantively deformed, gastrointestinal content enters the compartment 126 via the port 302. Consequently, the dissolvable block 604 starts to dissolve. When the dissolvable block 604 is substantively deformed, the twisted torsion-spring 602 starts to release the energy stored therein and applies a torque to the piston mandrel 144 to cause the piston mandrel 144 and the mucosal-layer brush 308 thereon to rotate, thereby providing an improved mucosal-lining-sampling performance.

FIG. 44 shows an ingestible apparatus 100, according to some embodiments of this disclosure. The ingestible apparatus 100 in these embodiments is similar to that shown in FIG. 43 except that the piston mandrel 144 is not rotatable. Rather, the mucosal-layer brush 308 is coupled to a sleeve 606 which is coupled to the piston mandrel 144 and is rotatable about a longitudinal axis thereof. The rotatable sleeve 606 is coupled to a torsion structure such as a twisted torsion-spring 602 retained in a dissolvable block 604 such that the torsion structure would not release the energy stored therein before the dissolvable block 604 is substantively deformed.

When the ingestible apparatus 100 is ingested in the GI tract and the shell 102 and coating 104 are substantively deformed, gastrointestinal content enters the compartment 126 via the port 302. Consequently, the dissolvable block 604 starts to dissolve. When the dissolvable block 604 is substantively deformed, the twisted torsion-spring 602 starts to release the energy stored therein and applies a torque to the rotatable sleeve 606 to cause the rotatable sleeve 606 and the mucosal-layer brush 308 thereon to rotate, thereby providing an improved mucosal-lining-sampling performance.

In above embodiments, the ingestible apparatus 100 comprises a dissolvable shell 102 coated with a dissolvable coating 104. In some alternative embodiments, the ingestible apparatus 100 does not comprise any dissolvable shell 102 or dissolvable coating 104.

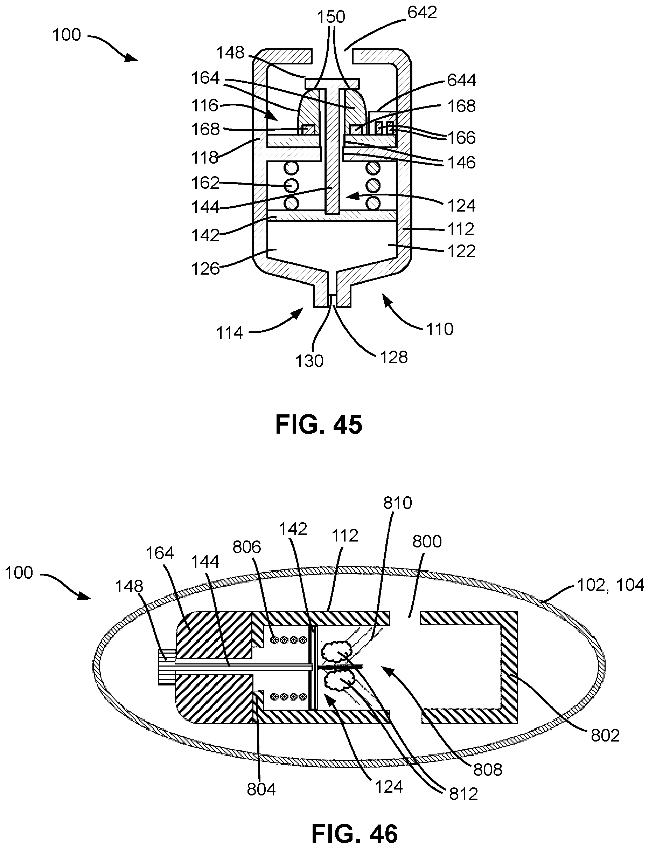

For example, in an embodiment shown in FIG. 45, the ingestible apparatus 100 only comprises a collection structure 110 which in this example is similar to the delivery structure 110 shown in FIG. 1A. However, for safety considerations, the housing 112 is further rearwardly extended to receive the entire piston 124 therein. The housing 112 also comprises an opening 642 about the rear end thereof to allow the gastrointestinal content to flow therein.

In this embodiment, the first set of electrodes 166 may be isolated from the gastrointestinal content by a dissolvable block 644.

When the ingestible apparatus 100 is ingested into a user's GI tract, gastrointestinal content flows into the rear portion of the housing via the opening 642 causing the dissolvable block 644 and the dissolvable latch 164 to dissolve. The dissolvable block 644 may be designed to dissolve faster than the dissolvable latch 164 in the gastrointestinal content. As a result, the first switch S₁ formed by the first set of electrodes 166 is first configured to a closed or on configuration after the dissolvable block 644 is substantively deformed, causing the transmission of a RF signal. Then, after the dissolvable latch 164 is substantively deformed, the piston 124 is actuated and the second switch S₂ (formed by the second set of electrodes 168 and the electrically conductive shoulder 150) is configured to a closed or on configuration thereby stopping the transmission of the RF signal.

Also shown in FIG. 45, all exterior edges and corners of the housing 112 are smooth or curved edges and corners for safety considerations. Similarly, the housing 112 in above-described embodiments and shown in FIGs. 1A to 44 may only comprise smooth or curved edges and corners for safety considerations.

In some embodiments, the circuit structure 118 of the ingestible apparatus 100 does not comprise a RF-signal transmitter. Rather, the circuit structure 118 comprises a timer and a memory. The circuit structure 118 uses the timer for recording the time instant of the occurrence of each state of the ingestible apparatus 100 and stores the recorded time instants in the memory. The stored time instants may be later retrieved for processing after the ingestible apparatus 100 is discharged from the user's body.

In some embodiments, the circuit structure 118 of the ingestible apparatus 100, such as the first and second detection circuits may be MEMS chips.

In an embodiment similar to that shown in FIG. 30, the dissolvable latch 164 is substantively deformed and the mucosal-layer brush 308 is extended out of the compartment 126 for collecting mucosal-cell samples, with or without discharging a health-related substance from the compartment 126. The method of containing and then extending the mucosal-layer brush with the action of the spring may be used to enact the outward extension of the mucosal-layer brush and enable the bristles to fully contact the mucosal lining.

In this embodiment, the mucosal-layer brush comprises bristles that are spring loaded and contained within the compartment 126. With extension of the piston, the bristles are allowed to extend radially outward beyond the edge of the housing to ensure contact with the mucosal lining.

In some embodiments, the mucosal-layer brush may comprise bristles which are formed from fluid-absorbent material such as hydrophilic fibers (e.g., poly-vinyl alcohol (PVA)). The fluid-absorbent material of the bristles may be used either to improve the collection of the gastrointestinal content or to deliver health-related substances to the mucosal layer through the bristles of the mucosal layer brush. Optionally, the bristles may change their properties with the absorbance of the fluid such that contact with the mucosal layer is further improved. For example, with contact with the gastrointestinal fluid the bristles may absorb fluid and become stiffer.

For example, FIG. 46 shows an ingestible apparatus 100 in some embodiments. The ingestible apparatus 100 in these embodiments comprises a dissolvable shell 102 and optionally an enteric-targeted coating 104 as described above. The dissolvable shell 102 receives therein a housing 112 having one or more openings 800 on one or more sidewalls thereof at a distance to a front wall 802.

A piston 124 movably extends into the housing 112 from a rear wall 804 thereof. The piston 124 comprises a piston plate 142 and a piston mandrel 144 extending rearwardly from the piston plate 142 out of the housing 112 to an end piece 148. The piston 124 engages a compressed spring 806 located in the housing 112 between the rear wall 804 and the piston plate 142.

At an initial state T_{A}, the piston 124 is located adjacent the rear wall 804 and is retained thereto by a dissolvable latch 164 sandwiched between and engaging with the end piece 148 and the rear wall 804 of the housing 112.At the initial state T_{A}, the piston plate 142 is located between the openings 800 and the rear wall 804.

A mucosal-layer brush 808 is coupled to the piston plate 142 on a side opposite to the side that the piston mandrel is coupled thereto. The mucosal-layer brush 808 comprises a plurality of bristles 810 and one or more fluid-absorbent materials 812 folded or otherwise compacted in the housing 112 at a location between the openings 800 and the piston plate 142.

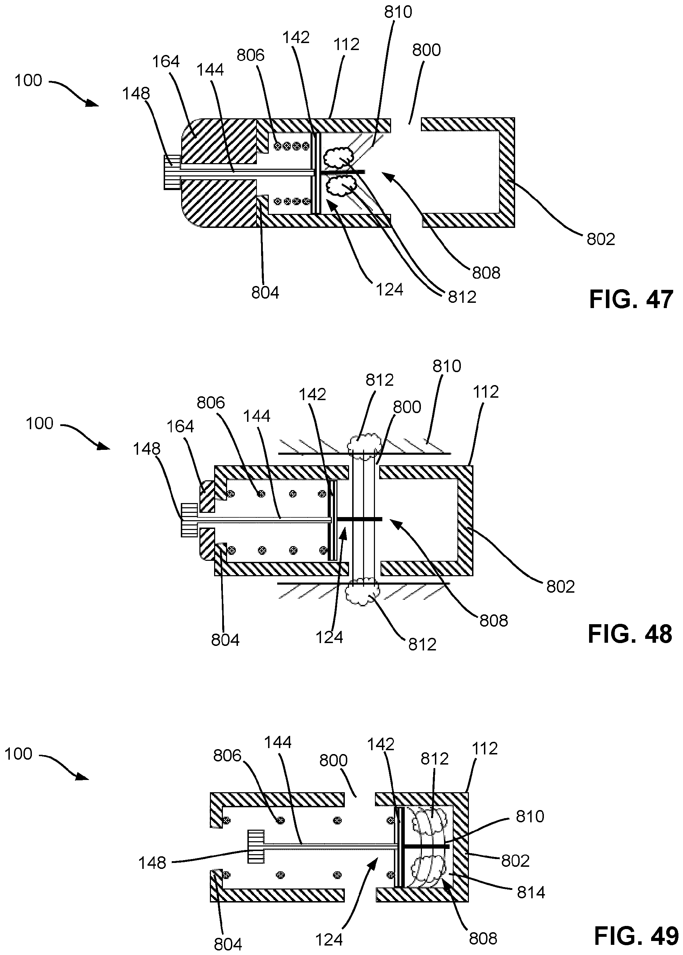

In a sampling process, the ingestible apparatus 100 may be ingested by a user. When the ingestible apparatus 100 arrives the target area, the dissolvable shell 102 and the enteric-targeted coating 104 are dissolved, as shown in FIG. 47.

Then, the dissolvable latch 164 is in contact with the gastrointestinal content and starts to dissolve. As shown in FIG. 48, with the dissolution of the latch 164, the spring 806 pushes the piston 124 towards the openings 800 and the front wall 802. As a result, the compacted bristle 810 and fluid-absorbent materials 812 are radially or laterally extended via the openings 800 out of the housing 112 and unfolded or otherwise fully expanded to collect the gastrointestinal content.

As shown in FIG. 49, the spring 806 continues to push the piston 124 towards the front wall 802 with the dissolution of the latch 164. The bristles 810, fluid-absorbent materials 812, and the gastrointestinal samples collected thereon (not shown) are then retracted into the housing 112. When the piston plate 142 passes the openings 800, the bristles 810, fluid-absorbent materials 812, and the gastrointestinal samples collected thereon are then sealed in the sealed compartment 814 between the front wall 802 of the housing 112 and the piston plate 142.

In some embodiments wherein the ingestible apparatus 100 comprises a mucosal-layer brush initially received in the housing 112 (e.g., in the embodiments similar to those shown in FIGs. 28 and 46), health-related substance (such as health-related substance in a powder form) may be initially attached to the bristles of the mucosal-layer brush. When the mucosal-layer brush is extended out of the housing 112 as described above, the health-related substance attached thereon is also extended out of the housing, thereby being delivered (or "painted") into the target area.

In some embodiments wherein the ingestible apparatus 100 comprises a mucosal-layer brush outside the housing and initially within the dissolvable shell 102 (e.g., in the embodiments similar to those shown in FIGs. 7, 43, and 44), health-related substance (such as health-related substance in a powder form) may be initially attached to the bristles of the mucosal-layer brush. When the dissolvable shell 102 is dissolved as described above, the health-related substance attached on the bristles of the mucosal-layer brush may be delivered (or "painted") into the target area.

Although embodiments have been described above with reference to the accompanying drawings, those of skill in the art will appreciate that variations and modifications may be made without departing from the scope thereof as defined by the appended claims.

## Claims

1. An ingestible apparatus (100) for collecting samples from a target area of a gastrointestinal (GI) tract of a human or mammal and/or for delivering a health-related substance thereto, the ingestible apparatus comprising:
a housing (112) comprising at least one first port (302 or 304) and a first compartment (122) communicable with the at least one first port;
an actuation assembly for collecting the samples and/or discharging the health-related substance through the at least one first port; and
a delay structure (164) for delaying the actuation assembly until the ingestible apparatus is within the target area;
**characterized in that** the actuation assembly comprises a brush (308), the brush comprising at least a stem and bristles coupled to the stem about a distal end thereof,
wherein the brush is configured to retract into the first compartment, and
wherein the bristles are configured to extend radially outward from the stem.

2. The ingestible apparatus of claim 1, wherein the bristles of the brush comprise a fluid- absorbent material.

3. The ingestible apparatus of claim 1 or 2, wherein the bristles of the brush are at least partially contained within the housing.

4. The ingestible apparatus of claim 3, wherein the actuation assembly is configured for extending the brush out of the housing.

5. The ingestible apparatus of claim 3, wherein the actuation assembly further comprises a biased spring (162).

6. The ingestible apparatus of claim 5, wherein the biased spring is configured for extending the brush out of the housing along a direction different to an orientation of the stem of the brush.

7. The ingestible apparatus of any one of claims 3 to 6, wherein the bristles of the brush are radially extendable beyond the edge of the housing.

8. The ingestible apparatus of claim 5, wherein the biased spring is configured to retract the brush into the first compartment.

9. The ingestible apparatus of claim 8, wherein the biased spring is a compressed or extended spring.

10. The ingestible apparatus of claim 9, wherein the compressed or extended spring is configured to become unbiased when the delay structure is dissolved by a GI content in the target area.

11. The ingestible apparatus of any one of claims 1 to 10, further comprising a stopper (306) for engaging the housing to close the at least one first port.

12. The ingestible apparatus of claim 11, wherein the stopper has a diameter greater than a diameter of the at least one first port.

13. The ingestible apparatus of any one of claims 1 to 12, wherein the brush is extendable out of the first compartment for collecting the samples and retractable into the first compartment for storing the samples.

14. The ingestible apparatus of claim 1, wherein the actuation assembly comprises:
a piston, at least a portion of the piston movably received in the housing; and
a first actuation structure for actuating the piston along a first direction from a first position to a second position, and wherein the delay structure comprises a latch retaining the piston in the first position, the latch dissolvable when contacting a GI content in the target area.

15. The ingestible apparatus of claim 1, wherein the actuation assembly is movable between a first position and a second position, and wherein the bristles are configured to spring out with a radial force in a direction different to the direction of the actuation assembly from the first position to the second position.

## Patentansprüche

1. Einnehmbare Vorrichtung (100) zum Sammeln von Proben aus einem Zielbereich eines Gastrointestinaltrakts (GI) eines Menschen oder Säugetieres und/oder zur Abgabe einer gesundheitsbezogenen Substanz dorthin, wobei die einnehmbare Vorrichtung umfasst:
ein Gehäuse (112) mit wenigstens einer ersten Öffnung (302 oder 304) und einem ersten Fach (122), das mit der wenigstens einen ersten Öffnung kommuniziert;
eine Betätigungsanordnung zum Sammeln der Proben und/oder zum Abgeben der gesundheitsbezogenen Substanz durch die wenigstens eine erste Öffnung; und
eine Verzögerungsstruktur (164) zum Verzögern der Betätigungsanordnung, bis sich die einnehmbare Vorrichtung innerhalb des Zielbereiches befindet;
**dadurch gekennzeichnet, dass** die Betätigungsanordnung eine Bürste (308) umfasst, wobei die Bürste wenigstens einen Stiel und Borsten umfasst, die um ein distales Ende des Stiels herum mit diesem verbunden sind,
wobei die Bürste so konfiguriert ist, dass sie sich in das erste Fach zurückzieht, und
wobei die Borsten so konfiguriert sind, dass sie sich von dem Stiel radial nach außen erstrecken.

2. Einnehmbare Vorrichtung nach Anspruch 1, wobei die Borsten der Bürste ein flüssigkeitsabsorbierendes Material umfassen.

3. Einnehmbare Vorrichtung nach Anspruch 1 oder 2, wobei die Borsten der Bürste zumindest teilweise in dem Gehäuse enthalten sind.

4. Einnehmbare Vorrichtung nach Anspruch 3, wobei die Betätigungsanordnung so konfiguriert ist, dass sie die Bürste aus dem Gehäuse herausfährt.

5. Einnehmbare Vorrichtung nach Anspruch 3, wobei die Betätigungsanordnung darüber hinaus eine vorgespannte Feder (162) umfasst.

6. Einnehmbare Vorrichtung nach Anspruch 5, wobei die vorgespannte Feder so konfiguriert ist, dass sie die Bürste in einer anderen Richtung als der Ausrichtung des Stiels der Bürste aus dem Gehäuse herausfährt.

7. Einnehmbare Vorrichtung nach einem der Ansprüche 3 bis 6, wobei sich die Borsten der Bürste radial über den Rand des Gehäuses erstrecken können.

8. Einnehmbare Vorrichtung nach Anspruch 5, wobei die vorgespannte Feder so konfiguriert ist, dass sie die Bürste in das erste Fach zurückzieht.

9. Einnehmbare Vorrichtung nach Anspruch 8, wobei die vorgespannte Feder eine komprimierte oder ausgezogene Feder ist.

10. Einnehmbare Vorrichtung nach Anspruch 9, wobei die komprimierte oder ausgezogene Feder so konfiguriert ist, dass sie nicht vorgespannt ist, wenn die Verzögerungsstruktur durch einen GI-Inhalt im Zielbereich aufgelöst wird.

11. Einnehmbare Vorrichtung nach einem der Ansprüche 1 bis 10, wobei die einnehmbare Vorrichtung darüber hinaus einen Stopfen (306) zum Eingriff in das Gehäuse umfasst, um die wenigstens eine erste Öffnung zu schließen.

12. Einnehmbare Vorrichtung nach Anspruch 11, wobei der Durchmesser des Verschlusses größer ist als der Durchmesser der wenigstens einen ersten Öffnung.

13. Einnehmbare Vorrichtung nach einem der Ansprüche 1 bis 12, wobei die Bürste zum Sammeln von Proben aus dem ersten Fach herausfahrbar ist und zum Aufbewahren der Proben in das erste Fach einziehbar ist.

14. Einnehmbare Vorrichtung nach Anspruch 1, wobei die Betätigungsanordnung umfasst:
einen Kolben, wobei wenigstens ein Teil des Kolbens beweglich im Gehäuse aufgenommen ist; und
eine erste Betätigungsstruktur zum Betätigen des Kolbens entlang einer ersten Richtung von einer ersten Position zu einer zweiten Position und wobei die Verzögerungsstruktur eine Verriegelung umfasst, die den Kolben in der ersten Position hält und die sich auflöst, wenn sie mit einem GI-Inhalt im Zielbereich in Berührung kommt.

15. Einnehmbare Vorrichtung nach Anspruch 1, wobei die Betätigungsanordnung zwischen einer ersten Position und einer zweiten Position beweglich ist, und wobei die Borsten so konfiguriert sind, dass sie mit einer radialen Kraft in einer anderen Richtung als die Richtung der Betätigungsordnung aus der ersten Position in die zweite Position herausspringen.

## Revendications

1. Appareil pouvant être ingéré (100) pour collecter des échantillons à partir d'une zone cible d'un tractus gastro-intestinal (Gl) d'un être humain ou d'un mammifère et/ou pour lui administrer une substance liée à la santé, l'appareil pouvant être ingéré comprenant :
un logement (112) comprenant au moins un premier orifice (302 ou 304) et un premier compartiment (122) pouvant communiquer avec l'au moins un premier orifice ;
un assemblage d'actionnement pour collecter les échantillons et/ou pour administrer la substance liée à la santé au travers de l'au moins un premier orifice ; et
une structure de retard (164) pour retarder l'assemblage d'actionnement jusqu'à ce que l'appareil pouvant être ingéré soit à l'intérieur de la zone cible ;
**caractérisé en ce que** l'assemblage d'actionnement comprend une brosse (308), la brosse comprenant au moins une tige et des poils qui sont couplés à la tige autour de son extrémité distale ;
dans lequel la brosse est configurée pour être rétractée à l'intérieur du premier compartiment ; et
dans lequel les poils sont configurés pour être étendus radialement vers l'extérieur depuis la tige.

2. Appareil pouvant être ingéré selon la revendication 1, dans lequel les poils de la brosse comprennent un matériau absorbeur de fluide.

3. Appareil pouvant être ingéré selon la revendication 1 ou 2, dans lequel les poils de la brosse sont au moins partiellement contenus à l'intérieur du logement.

4. Appareil pouvant être ingéré selon la revendication 3, dans lequel l'assemblage d'actionnement est configuré pour étendre la brosse en dehors du logement.

5. Appareil pouvant être ingéré selon la revendication 3, dans lequel l'assemblage d'actionnement comprend en outre un ressort sollicité par précontrainte (162).

6. Appareil pouvant être ingéré selon la revendication 5, dans lequel le ressort sollicité par précontrainte est configuré pour étendre la brosse en dehors du logement suivant une direction qui est différente d'une orientation de la tige de la brosse.

7. Appareil pouvant être ingéré selon l'une quelconque des revendications 3 à 6, dans lequel les poils de la brosse peuvent être étendus radialement au-delà du bord du logement.

8. Appareil pouvant être ingéré selon la revendication 5, dans lequel le ressort sollicité par précontrainte est configuré pour rétracter la brosse à l'intérieur du premier compartiment.

9. Appareil pouvant être ingéré selon la revendication 8, dans lequel le ressort sollicité par précontrainte est un ressort comprimé ou étendu par allongement.

10. Appareil pouvant être ingéré selon la revendication 9, dans lequel le ressort comprimé ou étendu par allongement est configuré pour devenir non sollicité par précontrainte lorsque la structure de retard est dissoute par un contenu de GI dans la zone cible.

11. Appareil pouvant être ingéré selon l'une quelconque des revendications 1 à 10, comprenant en outre un moyen d'arrêt (306) pour engager le logement pour fermer l'au moins un premier orifice.

12. Appareil pouvant être ingéré selon la revendication 11, dans lequel le moyen d'arrêt présente un diamètre qui est plus grand qu'un diamètre de l'au moins un premier orifice.

13. Appareil pouvant être ingéré selon l'une quelconque des revendications 1 à 12, dans lequel la brosse peut être étendue en dehors du premier compartiment pour collecter les échantillons et peut être rétractée à l'intérieur du premier compartiment pour stocker les échantillons.

14. Appareil pouvant être ingéré selon la revendication 1, dans lequel l'assemblage d'actionnement comprend :
un piston, au moins une section du piston étant reçue de façon mobile à l'intérieur du logement ; et
une première structure d'actionnement pour actionner le piston suivant une première direction depuis une première position jusqu'à une seconde position, et dans lequel la structure de retard comprend un moyen de blocage qui retient le piston dans la première position, le moyen de blocage pouvant être dissous lors de sa mise en contact avec un contenu de GI dans la zone cible.

15. Appareil pouvant être ingéré selon la revendication 1, dans lequel l'assemblage d'actionnement peut être déplacé entre une première position et une seconde position, et dans lequel les poils sont configurés de telle sorte qu'ils se dressent avec une force radiale dans une direction qui est différente de la direction de l'assemblage d'actionnement depuis la première position jusqu'à la seconde position.
